(19) Europäisches Patentamt
European Patent Office
Office européen des brevets

(11) Publication number: **0 297 913 B1**

# (12) EUROPEAN PATENT SPECIFICATION

(45) Date of publication of patent specification: **15.02.95**

(51) Int. Cl.[6]: **C12N 15/57**, C12N 9/64, C12N 5/00, //C12Q1/37

(21) Application number: **88306039.4**

(22) Date of filing: **30.06.88**

(54) **Production of Kallikrein.**

(30) Priority: **30.06.87 US 68594**
**27.06.88 US 210256**

(43) Date of publication of application:
**04.01.89 Bulletin 89/01**

(45) Publication of the grant of the patent:
**15.02.95 Bulletin 95/07**

(84) Designated Contracting States:
**AT BE CH DE ES FR GB GR IT LI LU NL SE**

(56) References cited:
**EP-A- 0 110 722**
**WO-A-87/02709**

**BIOCHEMISTRY, vol. 24, no. 27, December 1985, pp. 8037-8043, Washington, DC, US D. FUKUSHIMA et al.**

**CHEMICAL ABSTRACTS, vol. 105, December 22, 1986, page 338, abstracts 221500b, Columbus, Ohio, US A. IRIE et al.**

**CHEMICAL ABSTRACTS, vol. 87, no. 1, July 4, 1977, page 148, abstracts 1664c, Columbus, Ohio, US T. MODEER.**

(73) Proprietor: **AMGEN INC.**
**Amgen Center,**
**1840 Dehavilland Drive**
**Thousand Oaks, CA 91320-1789 (US)**

(72) Inventor: **Lin, Fu-Kuen**
**2491 Chaucer Place**
**Thousand Oaks, California 91360 (US)**
Inventor: **Lu, Hsieng Sen**
**3072 Chancery Place**
**Thousand Oaks, California 91362 (US)**

(74) Representative: **Brown, John David et al**
**FORRESTER & BOEHMERT**
**Franz-Joseph-Strasse 38**
**D-80801 München (DE)**

EP 0 297 913 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**THE JOURNAL OF BIOCHEMISTRY, vol. 104, no. 1, July 1988, pp. 22-29 Tokio, JP S: TAKAHASHI et al.**

**INT. J. PEPTIDE PROTEIN RES., vol. 33, 1989, pp.237-249, H.S. LU et al.**

**J. CLIN. ENDOCRIN. METAB., vol. 51, no.4, 1980, pp. 840-848, The Endocrine Soc., US K. SHIMAMOTO et al.**

## Description

The present invention relates to novel recombinantly derived glandular (tissue) kallikrein polypeptides and to methods for producing such polypeptides. The invention further relates to pharmaceutical compositions containing such polypeptides and to the use of such polypeptides and compositions as vasodilators and in the treatment of hypertension and male infertility.

### Background of the Invention

Kallikreins are members of a closely related subfamily of serine proteases. Kallikreins are characterized by their ability to release vasoactive peptides, kinins, from kininogen, although the physiological significance of proteolytic actions of these enzymes seems to be unrelated to the release of kinins at least in some instances, and certain kallikreins are thought to be involved in the specific processing for the generation of biologically active peptides as well as factors from their precursors. [Fukushima et al., Biochemistry 24, 8037-8043 (1985)].

The cDNA sequences for mouse and rat kallikrein were isolated from a submaxillary gland and a pancreatic cDNA bank. [Nakanishi et al., Biotechnology 3, 1089-1098 (1985)]. cDNA clones for human kallikrein were isolated from a human pancreatic cDNA bank [Fukushima et al., supra] and from a human kidney cDNA bank, [Baker et al., DNA 4, No. 6, 445-450 (1985)]. It was reported that the active enzyme form consists of 238 amino acids and is preceded by a signal peptide and profragment of 24 amino acids. It was also noted that the key amino acid residues required for serine proteinase activity (His-41, Asp-96, Ser-190) and for the kallikrein type cleavage specificity (Asp-184) are retained in the human kallikrein as they are in mouse and rat kallikreins.

### Summary of the Invention

In accordance with the present invention, a novel class of glandular kallikrein polypeptides is provided. These biologically active kallikrein polypeptides have the amino acid sequence extending from the N-terminus of the formula (I):

-1
(Ala Pro Pro Ile Gln Ser Arg)n

+1 10 20
Ile Val Gly Gly Trp Glu Cys Glu Gln His Ser Gln Pro Trp Gln Ala Ala Leu Tyr His

30 40
Phe Ser Thr Phe Gln Cys Gly Gly Ile Leu Val His Arg Gln Trp Val Leu Thr Ala Ala

50 60
His Cys Ile Ser Asp Asn Tyr Gln Leu Trp Leu Gly Arg His Asn Leu Phe Asp Asp Glu

70 80
Asn Thr Ala Gln Phe Val His Val Ser Glu Ser Phe Pro His Pro Gly Phe Asn Met Ser

90 100
Leu Leu Glu Asn His Thr Arg Gln Ala Asp Glu Asp Tyr Ser His Asp Leu Met Leu Leu (I)

110 120
Arg Leu Thr Glu Pro Ala Asp Thr Ile Thr Asp Ala Val Lys Val Val Glu Leu Pro Thr

130 140
Gln Glu Pro Glu Val Gly Ser Thr Cys Leu Ala Ser Gly Trp Gly Ser Ile Glu Pro Glu

150 160
Asn Phe Ser Phe Pro Asp Asp Leu Gln Cys Val Asp Leu Lys Ile Leu Pro Asn Asp Glu

170 180
Cys Lys Lys Ala His Val Gln Lys Val Thr Asp Phe Met Leu Cys Val Gly His Leu Glu

190 200
Gly Gly Lys Asp Thr Cys Val Gly Asp Ser Gly Gly Pro Leu Met Cys Asp Gly Val Leu

210 220
Gln Gly Val Thr Ser Trp Gly Tyr Val Pro Cys Gly Thr Pro Asn Lys Pro Ser Val Ala

230 238
Val Arg Val Leu Ser Tyr Val Lys Trp Ile Glu Asp Thr Ile Ala Glu Asn Ser

wherein n is 0 or 1.

The kallikrein polypeptides are characterized as the product of procaryotic or eucaryotic host expression (e.g., by bacterial, yeast, Bacillus and mammalian cells in culture) of exogenous DNA obtained by genomic, cDNA or by gene synthesis.

The DNA of the present invention includes DNA useful in securing expression in an appropriate host cell of a polypeptide product having the primary structural conformation of a kallikrein polypeptide having an amino acid sequence represented by formula (I) above and one or more of the biological properties of naturally occurring kallikrein. The DNA of the invention are specifically seen to comprise DNA encoding the sequence of formula (I) or their complementary strands. Specifically comprehended are manufactured DNA encoding kallikrein wherein such DNA may incorporate codons facilitating translation of messenger RNA in microbial hosts. Such manufactured DNA may readily be constructed according to the methods of Alton et al., PCT application WO 83/04053.

Also comprehended by the invention are pharmaceutical compositions comprising therapeutically effective amounts of the kallikrein polypeptide products of the invention together with suitable diluents, excipients and/or carriers useful in vasodilation and male infertility applications, etc.

The present invention also encompasses the various cloned genes, replicable cloning vehicles, expression vehicles and transformed cultures, all harboring the genetic information necessary to affect the production of recombinant derived kallikrein polypeptides of the present invention.

## Brief Description of the Drawings

Figure 1 illustrates the construction of pDSHK1.

Figure 2 is a photograph of a SDS-polyacrylamide gel comparing recombinant human mature kallikrein and naturally-occurring urinary kallikrein wherein the molecular weight markers are designated on the right-hand side and columns 1 and 2 designate urinary kallikrein and recombinant mature kallikrein under nonreducing conditions respectively, and columns 3 and 4 designate urinary kallikrein and recombinant mature kallikrein under reducing conditions.

Figure 3 illustrates the construction of pDGHK-L1A.

## Detailed Description of the Invention

According to the present invention, DNA sequences encoding the polypeptide sequence of human species glandular kallikrein of the present invention have been isolated and characterized. Further, the human DNA may be utilized in the eucaryotic and procaryotic expression providing isolatable quantities of polypeptides having biological and immunological properties of naturally-occurring kallikrein as well as in vivo and in vitro biological activities, in particular therapeutic activity, of naturally-occurring kallikrein.

The DNA of human species origin was isolated from a human genomic DNA library. The isolation of clones containing kallikrein encoding DNA was accomplished through DNA/DNA plaque hybridization employing a pool of mixed oligonucleotide probes.

The human kallikrein gene of the present invention encodes a 262-amino acid kallikrein polypeptide: a presumptive 17-amino acid signal peptide, a 7-amino acid proenzyme fragment and a 238-amino acid mature protein.

Procaryotic or eucaryotic host expression (e.g., by bacterial, yeast and mammalian cells in culture) of exogenous DNA of the present invention obtained by genomic or cDNA cloning or by gene synthesis yields the recombinant human kallikrein polypeptides described herein. The kallikrein polypeptide products of microbial expression in vertebrate (e.g., mammalian and avian) cells may be further characterized by freedom from association with human proteins or other contaminants which may be associated with kallikrein in its natural mammalian cellular environment or in extracellular fluids such as plasma or urine. The products of typical yeast (e.g., Saccharomyces cerevisiae) or procaryote (e.g., E. coli) host cells are free of association with any mammalian proteins. Depending upon the host employed, polypeptides of the invention may be glycosylated with mammalian or other eucaryotic carbohydrates or may be nonglycosylated. Polypeptides of the invention may also include an initial methionine amino acid residue (at position -1).

Illustrative of the present invention are cloned DNA sequences of monkey and human species origins and polypeptides suitably deduced therefrom which represent, respectively, the primary structural conformation of kallikrein of monkey and human species origins having the amino acid sequences represented by Table VIII.

The microbially expressed kallikrein polypeptides of the present invention may be isolated and purified by conventional means including, e.g., chromatographic separations or immunological separations involving monoclonal and/or polyclonal antibody preparations, or using inhibitors or substrates of serine proteases for affinity chromatography. Polypeptide products of the invention may be "labeled" by covalent association with a detectable marker substance (e.g., radiolabels, e.g., $I^{125}$ or nonisotopic labels, e.g., biotin) to provide reagents useful in detection and quantification of kallikrein in solid tissue and fluid samples such as blood or urine. DNA products of the invention may also be labeled with detectable markers (for example, radiolabels such as $I^{125}$ or $P^{32}$ and nonisotopic labels such as biotin) and employed in DNA hybridization processes to locate the kallikrein gene position and/or the position of any related gene family in the human, monkey and other mammalian species chromosomal map. The labeled DNA may also be used for identifying the kallikrein gene disorders at the DNA level and used as gene markers for identifying neighboring genes and their disorders.

The kallikrein polypeptide products provided by the invention are products having a primary structural conformation of a naturally-occurring kallikrein to allow possession of one or more of the biological properties thereof and having an average carbohydrate composition which may differ from that of naturally-occurring kallikrein.

Methods for administration of kallikrein polypeptide products of the invention include oral administration and parenteral (e.g., IV, IM, SC, or IP) administration and the compositions of the present invention thus administered would ordinarily include therapeutically effective amounts of product in combination with acceptable diluents, excipients or carriers. Therapeutically effective dosages are expected to vary substantially depending upon the condition treated and may be in the range of 0.1 to 100 $\mu$g/kg body weight of the active material. The kallikrein polypeptides and compositions of the present invention may also be lyophilized or made into tablets. Standard diluents such as human serum albumin are contemplated for pharmaceutical compositions of the invention, as are standard carriers such as saline.

The kallikrein products of the present invention may be useful, alone or in combination with other factors or drugs having utility in vasodilation and male infertility applications.

The following examples are presented by way of illustration of the invention and are specifically directed to procedures carried out prior to identification of kallikrein encoding monkey cDNA clones and human genomic clones, to procedure resulting in such identification, and to the sequencing, development of expression systems and immunological verification of kallikrein expression in such systems.

Numerous aspects and advantages of the invention will be apparent to those skilled in the art upon consideration of the following detailed description which provides illustrations of the practice of the invention in its presently preferred embodiments. As used herein in the following Examples, unless otherwise specified, the term recombinant kallikrein refers to recombinant mature kallikrein represented by the amino acid sequence of formula (I) wherein n is 0.

Example 1

Protein Sequence of Human Urinary Kallikrein

A. Amino Acid Sequencing of Human Urinary Kallikrein and Peptide Fragments

Human urinary kallikrein was purified from pooled urine of about 30 normal male Caucasian individuals according to the procedures described by J. Chao et al. [J. Clinical Endocrinology & Metabolism 51: 840-848 (1980)].

The pure human urinary kallikrein thus obtained was subject to N-terminus sequence analysis and the first 40 amino acids were identified. The purified urinary kallikrein protein was derivatized upon oxidation with performic acid and by reduced alkylation with dithiothreitol and iodoacetate. The protein derivatives were then digested using trypsin, Staphylococcus aureus SV-8 protease, and endolysine-C peptidase. Twenty-nine discrete peptide fragments were isolated from the digestions.

The peptide fragments thus derived from reduced and alkylated human urinary kallikrein were arbitrarily assigned numbers according to the protease used (i.e., T designates peptide fragments derived from trypsin digestion; S designates peptide fragments derived from SV-8 protease; and LC designates peptide fragments derived from endolysine C peptidase). The fragments were analyzed by microsequence analysis using a gas phase sequencer (Applied Biosystems), and the amino acid sequence of the human urinary kallikrein was determined and is represented in Table I. In addition the peptide fragments obtained from the above digests are also represented in Table I. In Table I, single letter designations are employed to represent the deduced translated polypeptide sequence of urinary kallikrein, an asterisk (*) designates unassigned amino acids, "NT" designates N-terminal sequencing of intact protein, "#" designates determined Asn-glycosylation site and " + " designates unassigned Asn-glycosylation site.

According to the isolation and sequence analysis of two overlapping peptides, S-18 and LC-17, represented in Table I, the amino acid at position 162 of human urinary kallikrein protein sequence was identified as lysine.

Table I

<u>Amino Acid Sequence of Urinary Kallikrein</u>

```
1                 10                  20                  30
I V G G W E C E Q H S Q P W Q A A L Y H F S T F Q C G G I L
|------------------------(NT)------------------------------
◄--S-38---►
◄-----S-41-----►
◄------------------------LC-64----------------------------
                  40                  50                  60
V H R Q W V L T A A H C I S D N Y Q L W L G R H N L F D D E
------------------------------------- * --- * * ------►
                      ◄-------T-50----------► ◄-----------

◄------------------------LC-64----------------------------
                  70                  80                  90
N T A Q F V H V S E S F P H P G F N M S L L E N H T R Q A D

-----------OX-T-43--------------- + --------- + ----►
◄-----S-37--------►
-------------------------LC-64---+-----------+-----------
                                    ◄------ + -LC-64-CBa-
                  100                 110                 10
E D Y S H D L M L L R L T E P A D T I T D A V K V V E L P T

                      ◄-------OX-T-33a--------► ◄----------
  ◄--S-7--► ◄-----------S-61------------► ◄---S-22--►
-----------------LC-64-----------------------► ◄----------
--------------► ◄---------LC-64-CBb-----------►

                  130                 140                 150
Q E P E V G S T C L A S G W G S I E P E N F S F P D D L Q C

-----------------T-62, T-58------------ # ----------------
        ◄------------S-52-------------►
--------------LC-54a, LC-51------------ # ----------------

                  160                 170                 180
V D L K I L P N D E C K K A H V Q K V T D F M L C V G H L E

------►                               ◄----OX-T-41, T-52a----
                  ◄---------S-18--------►
------► ◄-----LC-17-----►             ◄----------LC-45-------
                  190                 200                 210
G G K D T C V G D S G G P L M C D G V L Q G V T S W G Y V P
      ◄---------------------------T-53---------------------
----► ◄-----------------------------T-57-----------------

----► ◄--------------------------LC-50--------------------
      ◄------------------------------LC-54b----------------
                  220                 230
C G T P N K P S V A V R V L S Y V K W I E D T I A E N S
----------►
----------------------► ◄--T-29---► ◄------T-40-------►
                              ◄--S-45---►
----------► ◄--------LC-42--------► ◄-----LC-33-------►
----------------------------------►
```

This result is consistent with the amino acid sequence deduced from human genomic kallikrein DNA sequence as set forth in Table V. This result, however, is different from the reported sequence derived from human pancreatic cDNA and kidney cDNA, wherein glutamic acid was present at position 162 of the reported mature kallikrein, [Baker et al., DNA <u>4</u>, 445-450 (1985) and Fukushima et al., Biochemistry <u>24</u>, 8037-8043 (1985)].

B. Glycosylation Sites

It was determined that human urinary kallikrein contains approximately 30% carbohydrate content based on the molecular weight estimated by sodium dodecyl sulphate (SDS) polyacrylamide gel electrophoresis. The amino acid sequence of human urinary kallikrein indicates that there are three potential Asn-linked glycosylation sites. Sequence analysis of peptide fragments obtained in Example IA., indicates that there are three Asn-linked glycosylation sites (Table II). However, Asn 141 was found only partially glycosylated (60%). This is based on the sequencing results of two peptides containing identical sequence (T-58 & LC-51 vs. T-62 & LC-54A). Asn 141 when linked to carbohydrate, as in fragments T-62 and LC-54a, could not be identified by sequence analysis of the glycopeptide.

Table II

| Isolation and Characterization of Glycopeptides | | | |
|---|---|---|---|
| Fragments No. | Position | Asn-linked | Glycosylation site |
| LC-64 | 1-114 | yes | Asn 78; Asn 84 |
| T-58 LC-51 | 115-154 | yes | Asn 141 |
| T-62 LC-54a | 115-154 | no | no |

Example 2

Design and Construction of Oligonucleotide Probe Mixtures

The amino acid sequence set out in Table I was reviewed in the context of the degeneracy of the genetic codons for the purpose of ascertaining whether mixed probe procedures could be applied to DNA/DNA hybridization procedures on cDNA and/or genomic DNA libraries.

N-terminus amino acid residues 1-10 and 12-29 and Phe-Asp-Asp-Glu-Asn-Thr-Ala-Gln-Phe-Val fragment from a tryptic peptide fragment OX-T-43 (see Table I) were chosen for synthesis of deoxyoligonucleotide probe mixtures and the probe mixtures represented in Table III were designed:

## Table III

### Oligonucleotide Probes

| Probe | | 1 | 2 | 3 | 4 | 5 | 6 | 7 | 8 | 9 | 10 | |
|---|---|---|---|---|---|---|---|---|---|---|---|---|
| | | Ile | Val | Gly | Gly | Trp | Glu | Cys | Glu | Gln | His | |
| HK-1a | 5' | ATT | GTG | GGC | GGA | TGG | GAA | TGC | GAA | CAA | CA | 3' |
| | | | | | G | | G | T | G | G | | |
| HK-1b | 5' | ATT | GTG | GGC | GGC | TGG | GAA | TGC | GAA | CAA | CA | 3' |
| | | | | | T | | G | T | G | G | | |

| Probe | | 12 | | | | | | | | 20 | | | | | | | | | 29 | |
|---|---|---|---|---|---|---|---|---|---|---|---|---|---|---|---|---|---|---|---|---|
| | | GLN | PRO | TRP | GLN | ALA | ALA | LEU | TYR | HIS | PHE | SER | THR | PHE | GLN | CYS | GLY | GLY | ILE | |
| HK-6a | 3' | GTT | GGI | ACC | GTT | CGT | CGG | GAC | ATG | GTG | AAG | AGI | TGI | AAG | GTC | ACA | CCI | CCI | TA | 5' |
| | | C | | | C | | | | A | A | A | | | | | | | | | |
| HK-6b | 3' | GTT | GGI | ACC | GTT | CGA | CGG | GAC | ATG | GTG | AAG | AGI | TGI | AAG | GTC | ACA | CCI | CCI | TA | 5' |
| | | C | | | C | | | | A | A | A | | | | | | | | | |
| HK-7a | 3' | GTT | GGI | ACC | GTT | CGA | CGG | GAC | ATG | GTG | AAG | TCG | TGI | AAG | GTC | ACA | CCI | CCI | TA | 5' |
| | | C | | | C | | | | A | A | A | | | | | | | | | |
| HK-7b | 3' | GTT | GGI | ACC | GTT | CGT | CGG | GAC | ATG | GTG | AAG | TCG | TGI | AAG | GTC | ACA | CCI | CCI | TA | 5' |
| | | C | | | C | | | | A | A | A | | | | | | | | | |

(I = Inosine)

| Probe | | | | | | | | | | | | |
|---|---|---|---|---|---|---|---|---|---|---|---|---|
| | | PHE | ASP | ASP | GLU | ASN | THR | ALA | GLN | PHE | VAL | |
| KF-2b | 5' | TTC | GAC | GAC | GAA | AAC | ACT | GCC | CAG | TTT | GT | 3' |
| | | T | T | T | G | T | | | | | | |

The probe mixtures HK-1a and 1b are mixtures of 64 probes 29 nucleotides in length and HK-6a, 6b, 7a and 7b are mixtures of 32 probes, 53 nucleotides in length and probe KF-2b is a mixture of 32 probes, 29 nucleotides in length.

The oligonucleotide probes were labeled at the 5′ end with γ- $^{32}$P-ATP, 7500-8000 Ci/mmole (ICN) using $T_4$ polynucleotide kinase (NEN).

Example 3

Hybridization

Probes HK-1a, 1b, 6a, 6b, 7a and 7b were used to hybridize human DNA Southern blots or monkey kidney poly(A)$^+$ mRNA blot to determine specific hybridization and for defining the hybridization conditions. Probe mixtures HK-1b at 45°C and HK-7b at 46°C yielded specific hybridization in a hybridization buffer comprising 0.025 pmol/ml of each of the probe sequences in 0.9 M NaCl/5 mM EDTA/50 mM sodium phosphate, pH 6.5/0.5% sarkosyl/100 $\mu$g of yeast tRNA per ml. As a result, these two sets of probe mixtures were employed in the monkey kidney cDNA library screening described in Example 4C.

Example 4

A. Monkey cDNA Library Construction

A monkey kidney cDNA library was constructed from poly(A)$^+$ mRNA isolated from anemic cynomolgus monkey kidneys as described in PCT Patent Application No. WO 85/02610 and Lin et al., [Gene 44: 201-209, (1986)]. Messenger RNA was isolated from anemic monkey kidneys by the guanidinium thiocyanate procedure of Chirgwin et al., [Biochemistry 18: 5294-5299 (1979)] and poly(A)$^+$ mRNA was purified by two runs of oligo(dT)-cellulose column chromatography as described by Maniatis et al., ["Molecular Cloning, A Laboratory Manual", p. 197-198 Cold Springs Harbor Laboratory, Cold Springs Harbor, NY, (1982)]. The cDNA library was constructed according to a modification of the general procedures of Okayama et al. [Mol. and Cell Biol. 2, 161-170 (1982)]. The procedures are summarized as follows: (1) pUC8 was used as the sole vector, cut with PstI and then tailed with oligo dT of 60-80 bases in length; (2) HincII digestion was used to remove the oligo dT tail from one of the vector; (3) first strand synthesis and oligo dG tailing was carried out according to the Okayama procedure; (4) BamHI digestion was employed to remove the oligo dG tail from one end of the vector; and (5) replacement of the RNA strand by DNA was in the presence of two linkers (GATCTAAAGACCGTCCCCCCCCC and ACGGTCTTTA) in a three-fold molar excess over the oligo dG tailed vector.

B. Bacterial Transformation

Transformation of DNA into E. coli strain DH1 was performed and transformants were selected on LM-Ap agar containing 1% (w/v) Bacto tryptone/0.5% (w/v) yeast extract - 10 mM NaCl-10 mM $MgSO_4$, 1.5% (w/v) Bacto agar containing 50 $\mu$g ampicillin per ml. [Hanahan, J. Mol. Biol. 166: 557-580 (1983)]. Transformants were obtained at a level of 1.5 x $10^5$ per $\mu$g of poly(A)$^+$ RNA.

C. Colony Hybridization Procedures for Screening Monkey cDNA Library

The colony hybridization procedures employed for screening the monkey cDNA library were essentially the same as described in PCT application No. WO 85/02610 and Lin et al. [Gene 44: 201-209, (1986)].

Transformed E. coli were spread out at a density of 9000 colonies per 10 x 10 cm plate on nutrient plates containing 50 $\mu$g of ampicillin per ml. Gene Screen filters (New England Nuclear Catalog No. NEF-972) were pre-wet on a BHI-CAM plate (Bacto brain heart infusion 37 g/L, casamino acids 2 g/L and agar 15 g/L, containing 500 $\mu$g of chloramphenicol/ml) and were used to lift the colonies off the plate. The colonies were grown in the above medium for 12 hours or longer to amplify the plasmid copy numbers. The amplified colonies (colony side up) were treated by serially placing the filters over 2 pieces of Whatman 3 MM paper saturated with each of the following solutions:

(1) 50 mM glucose - 25 mM Tris-HCl (pH 8.0) - 10 mM EDTA (pH 8.0) for five minutes;
(2) 0.5 M NaOH for ten minutes; and
(3) 1.0 M Tris-HCl (pH 7.5) for three minutes

The filters were air-dried in a vacuum oven at 80°C for two hours and then treated with a solution containing 50 $\mu$g of proteinase K per ml. in Buffer K, which contains 0.1 M Tris-HCl (pH 8.0), 0.1 M NaCl, 10 mM EDTA (pH 8.2) and 0.2% sarkosyl. Specifically, 5 ml of the proteinase K solution was added to each filter and the digestion was allowed to proceed at 55°C for 30 minutes, after which the solution was removed.

The filters were treated with 4 ml of a prehybridization buffer [5 x SSPE ( 0.1M NaCl/5mM EDTA/ 50mM Na Phosphate,pH 6.5) - 0.5% Sarkosyl - 100 $\mu$g/ml single stranded E. coli DNA - 5 x BFP (1 x BFP = 0.02% wt./vol. of BSA, Ficoll (M.W. 400,000) and Polyvinylpyrrolidone)]. The prehybridization treatment was carried out at 55 °C, generally for 4 hours or longer, after which time the prehybridization buffer was removed.

The hybridization process was conducted as follows: To each filter was added 3 ml of hybridization buffer (5 x SSPE - 0.5% sarkosyl - 100 µg of yeast tRNA per ml) containing 0.075 picomoles of each of the 64 probe sequences of Table III (the total mixture being designated a HK-1b) and the filters were maintained at 45 °C for 20 hours.

Following hybridization, the filters were washed three times for ten minutes on a shaker with 6 x SSC (1 x SSC = 0.15 M NaCl, 15 mM sodium citrate, pH 7) - 0.5% sarkosyl at room temperature and washed two to three times with 6 x SSC - 1% sarkosyl at the hybridization temperature (45 °C), then autoradiographed. The filters were incubated at 100 °C in 1 x SSC, pH 7.0/0.1% sarkosyl for 2 min. to remove the hybridized probes. The filters were again prehybridized as described above and then hybridized with the HK-7b mixed probes at 46 °C and washed as described above.

Four positive clones that were hybridized to both HK-7b and HK-1b probe mixtures were obtained among the 200,000 colonies screened and were further confirmed by hybridization to another set of probe mixture KF-2b at 48 °C.

One of the positive clones designated as MKK80a was further analyzed and sequenced by the dideoxy method of Sanger et al., [Proc. Natl. Sci. Acad., USA, 74: 5463-5467 (1977)]. The nucleotide sequence of MKK80a clone insert is depicted in Table IV (wherein the arrow "↓" designates the beginning of the monkey kallikrien sequence), which is 95% homologous to that of the coding region of the human genomic kallikrein clone λHK65a as shown in Table V. The amino acid sequence deduced from the nucleotide sequence of the monkey clone MKK80a exhibited 93% homology to that of human kallikrein as illustrated in Table VIII.

# Table IV

## MKK80a Clone Nucleotide Sequence

```
                10                  20                  30               40                  50                  60
GA ATT CCC GGG GAT CTT AAA GAC CGT CCC CCC CCC CCC AGC TCC TCC ACC TGC CGG CCC CTG

            70                  80                  90                 100                 110                 120
GAC ACC TCT GTC ATC ATG TGG TTC CTG GTT CTG TGC CTC GCC CTG TCC CTG GGG GGG ACT
                    Met Trp Phe Leu Val Leu Cys Leu Ala Leu Ser Leu Gly Gly Thr

           130                 140                 150                 160                 170                 180
GGT CGT GCG CCC CCG ATT CAG TCC CGG ATT GTG GGA GGC TGG GAG TGT TCC CAG CCC TGG
Gly Arg Ala Pro Pro Ile Gln Ser Arg Ile Val Gly Gly Trp Glu Cys Ser Gln Pro Trp

           190                 200                 210                 220                 230                 240
CAG GCG GCT CTG TAC CAT TTC AGC ACT TTC CAG TGT GGG GGC ATC CTG GTG CAT CCC CAG
Gln Ala Ala Leu Tyr His Phe Ser Thr Phe Gln Cys Gly Gly Ile Leu Val His Pro Gln

           250                 260                 270                 280                 290                 300
TGG GTG CTC ACA GCT GCC CAT TGC ATC AGC GAC AAT TAC CAG CTC TGG CTG GGT CGC CAC
Trp Val Leu Thr Ala Ala His Cys Ile Ser Asp Asn Tyr Gln Leu Trp Leu Gly Arg His

           310                 320                 330                 340                 350                 360
AAC TTG TTT GAT GAC GAA GAC ACA GCC CAG TTT GTT CAT GTC AGT GAG AGC TTC CCA CAC
Asn Leu Phe Asp Asp Glu Asp Thr Ala Gln Phe Val His Val Ser Glu Ser Phe Pro His

           370                 380                 390                 400                 410                 420
CCT GGC TTC AAC ATG AGC CTC CTG AAG AAC CAC ACC CGC CAA GCA GAT GAT TAC AGC CAC
Pro Gly Phe Asn Met Ser Leu Leu Lys Asn His Thr Arg Gln Ala Asp Asp Tyr Ser His

           430                 440                 450                 460                 470                 480
GAC CTC ATG CTG CTC CGC CTG ACG CAG CCT GCC GAG ATC ACA GAC GCT GTG CAG GTC GTG
Asp Leu Met Leu Leu Arg Leu Thr Gln Pro Ala Glu Ile Thr Asp Ala Val Gln Val Val

           490                 500                 510                 520                 530                 540
GAG TTG CCC ACC CAG GAA CCC GAA GTC GGG AGC ACC TGT TTG GCC TCC GGC TGG GGC AGC
Glu Leu Pro Thr Gln Glu Pro Glu Val Gly Ser Thr Cys Leu Ala Ser Gly Trp Gly Ser

           550                 560                 570                 580                 590                 600
ATC GAA CCA GAG AAT TTC TCA TTT CCA GAT GAT CTC CAG TGT GTA GAC CTC GAA ATC CTG
Ile Glu Pro Glu Asn Phe Ser Phe Pro Asp Asp Leu Gln Cys Val Asp Leu Glu Ile Leu

           610                 620                 630                 640                 650                 660
CCC AAT GAT GAG TGC GCC AAA GCC CAT ACC CAG AAG GTG ACA GAG TTC ATG CTG TGT GCC
Pro Asn Asp Glu Cys Ala Lys Ala His Thr Gln Lys Val Thr Glu Phe Met Leu Cys Ala

           670                 680                 690                 700                 710                 720
GGA CAC CTG GAA GGT GGC AAA GAC ACC TGT GTG GGT GAT TCA GGG GGC CCG CTG ACG TGT
Gly His Leu Glu Gly Gly Lys Asp Thr Cys Val Gly Asp Ser Gly Gly Pro Leu Thr Cys

           730                 740                 750                 760                 770                 780
GAT GGT GTG CTC CAA GGT GTC ACA TCA TGG GGC TAC ATC CCT TGT GGC AGC CCC AAT AAG
Asp Gly Val Leu Gln Gly Val Thr Ser Trp Gly Tyr Ile Pro Cys Gly Ser Pro Asn Lys

           790                 800                 810                 820                 830                 840
CCT GCT GTC TTC GTC AAA GTG CTG TCA TAT GTG AAG TGG ATC GAG GAC ACC ATA GCG GAG
Pro Ala Val Phe Val Lys Val Leu Ser Tyr Val Lys Trp Ile Glu Asp Thr Ile Ala Glu

           850                 860                 870                 880                 890                 900
AAC TCC TGA ATG CCC AGC CCC GTC CCC TAC CCC CAG TAA AAT CGA ATG TGC ATC AAA AAA
Asn Ser ---

           910                 920
AAA AAA AAA AAA AAA AAA AAA AA
```

Example 5

Phage Plaque Hydridization Procedures for Isolating Kallikrein Gene From the Human Genomic Library

A Ch4A phage-borne human fetal liver genomic library prepared according to the procedures described by Lawn et al., [Cell 13: 533-543 (1979)] was obtained and used in a plaque hybridization assay. The phage plaque hybridization procedures employed were as described in PCT No. WO 85/02610 and Lin et al., [Proc. Natl. Sci. Acad., USA 82: 7580-7584 (1985)]. Phage plaques were amplified according to the procedures of Woo, [Methods Enzymol. 68, 389-395 (1979)], except that Gene Screen Plus filters and NZYAM plates [NaCl, 5 g; $MgCl_2.6H_20$, 2 g; NZ-Amine A, 10 g; yeast extract, 5 g; casamino acids, 2 g; maltose, 2 g; and agar, 15 g (per liter)] were utilized. Phage particles were disrupted by alkali treatment and the DNA's were fixed onto filters (50,000 phage plaques per 8.4 x 8.4 cm filter). The air-dried filters were baked at 80 °C for 1 hour and then subjected to proteinase K digestion as described in Example 4. Prehybridization with a 1 M NaCl/1% sarkosyl solution was carried out at 55 °C for 4 hours or longer.

The monkey kallikrein MMK80a clone DNA was nick-translated with $^{32}P$-labeled-$\alpha$dCTP, and the cDNA insert (~1000 bp) was cut out by double-digestions with EcoRI plus HindIII and used as a probe to screen human fetal liver genomic library.

The hybridization buffer contained 2 $x10^5$ cpm/ml of the nick-translated monkey kallikrein cDNA in 0.9 M NaCl/5 mM EDTA/50 mM sodium phosphate, pH 6.5/0.5% sarkosyl/100 $\mu$g of yeast tRNA per ml. Hybridization was carried out at 55 °C for 20 hours. At the completion of hybridization, the filters were washed three times with 6 x SSC, pH 7.0/0.5% sarkosyl at room temperature and two times at the hybridization temperature, 10 min. per wash.

Two strongly positive clones, designated as λHK65a and λHK76a, were obtained among a total of 1.87 x $10^6$ phage plaques screened.

Both human genomic kallikrein lambda clones were subcloned into pUC118 or pUC119 and the double-stranded DNA's were sequenced according to the procedure of Chen et al., [DNA 4, 165-170 (1985)] using the dideoxy method of Sanger et al., [Proc. Natl. Acad. Sci. USA 74: 5463-5467 (1977)], and the nucleotide sequence of kallikrein gene containing region for clone λHK65a is represented in Table V.

## Table V

## Clone λHK65a Genomic Sequence

```
AGATCTCTGGGACTTTGGAGGACAGGACGTTGAGCACCCATAACTGGGAGCAAGAAGTCCAGGCTCATTGAGACCCCAGCATTGTTTGGGAGGGACACTGGATTTTCTAATGCTAAAGAACTTACCCTGGGAGACCCCT  140
GAAACTTCACATTCCTGGATCTGGAGGCACTAGGAATGTGGTGATGTGTACCCCAAAAT..TATTATGAAAGGAACAGTAGCTTTGGGGACCTAGGTCCTGGGTCTGGGAGTGCAAGAACCAGGGCATTCTGAAGGCCAA  280
GGCTTATATTTCAGGGGGAACTGGCAGCTGAGATGCTAGTATCTTGGCTCCGAGTGGTGCAGGGAATCAGAGCCCTAACTGCTGGTGTTTGAGGGCACCAGGTCTGGGGGTGCAGGGCCTTAATTGAGACTGAGGTATAT  420
GGGGCTTTGTACCAGGGGTCACTGATTCCTCCGTCTTCCTGTTTCTGCTTCCCCTCAGTCCCCCCTTGCCTCACTGGCTGCTCCCCAGAGCAAAGCCCCAGAGCCACAGGCCCTGCCCCACCTGGCTTCAACCCCAGGAA  560
TGCGTCCAGCGTGATCCAGGGCCTGCAGAACAGGTGCTGGTTCTCCCTCCCCGTCTACAGCTCTGGGGATCGGAGGCGGGGGGGCAATTGTCCAGCCCCCAGGCAACAGGGGAAGGGCCTTGGCAACATCTGGGTGCCAG  700
                                                                                                       -24         -20
                                                                                                       MetTrpPheLeuValLeuCysLeuAlaLeuSerLeuGl
CAGGGCAGGGGTGGGGCTCTGAGGGGATAAGGGCTTTTAAAAGCCTCCCCAGGGAGGTTCCCCAGTTCCTCCACCTGCTGGCCCCTGGACACCTCTGTCACCATGTGGTTCCTGGTTCTGTGCCTCGCCCTGTCCCTGGG  840
   -10
yGlyThrG
GGGGACTGGTGAGACAGTGGGGGGATGTGGAGGGGGAACGGGCCCTGATTCTCTTGCTGGCCTCACATCCCCCCCCGATAAGACCTTCCCCTCCAACACCCCACCCTCATCCCTCTGGCCCACAGTCTATCCTAGTCCG  980
GGGTGCCCCGGCTCTTATCTATCTTGCGCGGCCCCACCCTAAACTTCCACCCCCGCTCCTCCATGTTGTCATAGTGCTCACTCCCACACCAGATTCCTGCTCCTCCCAGGAAGCCTCAGTACTTTCTCCTCTTCCCTAGC  1120
CAACGCCCTGTCCCACCGCTTTACCAAAGGGGCAGATTCTGGGCCATCTCTGTCTCTCTCTGGAGGACCCCAACCCTCCCATGGGCTTCCCCCACCCCACAGGACTCTGATCATCCCCTCCTGCCCCCTAGTTCCCTGCG  1260
GCCACCTATTTGGCTCCTGAGTCTACAAATCCCACCACACTCCAGTGACTTTTTCCATCCAGCTGTCCGCTCTCAACTGTGTCCTCCAGACCCTGACATCTGGTCTCCCCAGTCCTTCCAGGGTCCCCCAAATTTCACCA  1400
GAAAACCCTGCTTCCCAAACTGGCATGTGGGATCCCCCATCTGGTATGGGGGGGGGTCTGCAAATGTCCCTTCCCACCCAGCACCCCCAGCTCTCCCAGGAAAAACACCACACCCTCCCACCACATGCTCCTTTCCTTGG  1540
GGAAGTTCCATCCCGCCCCAGGCCCCAGCCCTTCTCTGCCTGAATTGTTCCTACACAGCTCTGGTTTTCTGGAACCCCACGCCAAGGATCCTGAATGCCTTCTCAGCCAGCGCTTCTGCCCTGGCTCCCCCAAGTCAGTA  1680
TGTGATTCTACAACCTCACATCAAGTGGGTCCTGCATTGCATTCCCACAGCTGCTTGGCGCCTGTTCTCACCACTTCTCCAATCCTCTTCCTAGGATTGCCTCCAAGAGGACCCCATATCCTTCCAGTACCCTCAGACCA  1820
TGAACCCCATCCCCATGAGAAACGAAGATCCCATCCTTCACCCTGTCCGAGAAGGACTGCCACTCACGCCTCCCTCCTCCTGAGCCCCGTCCCAGTCCTGACCGCAACTGTGTTCTCAGGGCCCCAACCTCCCCTCATAT  1960
TGCCATTCCTGGTTCACAGGAAGGACCCCAAAACCTCTCGGAAGCCCACCTCTAGCCTTAACCATAGCCCTTTCTTTTACCCTTTAACCAGGAAGCCCTGAAATCTAACTGTCCAGCCAAGCTGGCCCCTTCCAACCCCA  2100
ATGTAGGGACCCCAAGTCACCCCCAAACTCTTCAGTCGAGATGCTCTTCTTTACCTCTCAGACCTGGTTCCCTAAAGCTGCATCCTTTCCTGAACCTCAAAAGCCCAACCCAGGTCCTGTTTCCCTAGCCTAGGAGCCCC  2240
TTAGCCCTCCAGATTCTTCCTCAGTGAGCCTCTGATCCTGTCCCACCCTTGGCGCCTTGATCCCTGGTTTCCAGAATCCCTTTCCACTCCCCTAACCCACCACCTCCCATCCCCCAGCCCCAGACCCCAAATGACCTGAC  2380
GCCCAGATGCAGTGTCTGCTCCAGACACTGTCTCCTGTCTCCTACCCTGATCCCTGGAGGCTGCTCTACTGTCAGAGCACAAAATCTCCCCACCAGGTCCAGCCACCACTCCAAAAGCCCAGGGAACAGTCCAGAGTCCC  2520
ACCCCTTCCCAACACCCCTTGCCCATGTCCCCAACCTCCAGCCCTGGTCCTCTACCCGCAATGTCCCTTCAGACCCACAGCCCAGCTCCCTCTCCTAGCCTTGTCCCTGGCCTCTCCTGCCAACCCTGCCCTTCCTGACC  2660
                                      -1  1                    10                         20                        30
                     lyAlaAlaProProIleGlnSerArgIleValGlyGlyTrpGluCysGluGlnHisSerGlnProTrpGlnAlaAlaLeuTyrHisPheSerThrPheGlnCysGlyGlyIleLeuValHisA
CAGCACCGCCTCTGCAGGTGCTGCGCCCCCGATTCAGTCCCGGATTGTGGGAGGCTGGGAGTGTGAGCAGCATTCCCAGCCCTGGCAGGCGGCTCTGTACCATTTCAGCACTTTCCAGTGTGGGGGCATCCTGGTGCACC  2800
                     40
rgGlnTrpValLeuThrAlaAlaHisCysIleSerAs
GCCAGTGGGTGCTCACAGCTGCTCATTGCATCAGCGAGTGAGTAGGGGCCTGGGGTCTGGGAGGAGGGCATCTATGCTGCCACAGGATTGACAGGCCAGTGGCATTCCCCTTGGATAACCTCAGGCAAGACTGGGGGGCT  2940
GAGCCAGAGAGGAAGGCTCTGGCGCAGGTCGCCTGGCAGGGCAGAGCTGGGCTGGACACCCCTCTCCAAGGCTGCCTGGGTCTCTTGGGATCTGGTTCTGCTTGTGTCTCTGTGTGACTGTGTTCTGGTCTCTGTCTTCC  3080
TCTCTCTCCTCTGTCTCCTTGTCTCTGCATCTCCCCTGTCTCTGTCTGTCTCTGAGTCTCTCTGCGGCATCTCTGTCACTGTGTCTCACCCTCCATCTCTCTACCCATCTCTCTCTCTCTGGGTCTCTACCTCACCGCTC  3220
CCTCATCCCTACTAAACACACACCCAGATGGACCTAAGGGAGGCCCCAGAGTAAAGGAAGGGCTTTATCCCCAACTTGTGTGCCTGGGAGGGGGGCGCCTCTCCCTCTGTCCAGCTCCCCGCCCCACAGGATATCCCTCCA  3360
CTCTGGAGAGACACAGGGAAGGGCTGGTTTCAGCGGGAGCTGGGTGGGGCAACTGAGGGAGGAGGAAGGAGGAGGCGGGGCGAGAAACGCGCGGGAGGGTGCTGGGAACCGAAGGGGGCCTCGACCTTGGACTTCAGGCC  3500
AGGCCACCTGCCCCTGGGGAGCCCGCACCACAGCCCCAGCTGCAGCTGAGCCGCTCTCAGGCCTCCCCTCCTCCCTACCTGCCCCCACCCTCCCCACCTGCCCCCACCCTCCCCCAGGGCCTCCCTCCCTTTTCTCTCCC  3640
ACACTCGGTCACTCCTGCTTCCTCTCTGCCTCTGTTTCTAGTTCTCACTCTGACGTCCCGCGTCCTTTTTCATTTGTCTGCTTCTCGCTCCCTTCCGTGTTCTGTTCCCTCTTCCTCCCTCTTCCCTGGGGCCTGTTTCT  3780
CGCTGTCCCTGTCTTTTATCCTTCTCATCTGCCTCTTTTTTGCTCACCCTGTTTCTCACTGCCCTCCCCTCCGCCTTTCTCACCCCTCTGTCCTTTTGAGCTCTTTTTTGCTCTGTCTCTTCATTTGCCTTTTGCACTCT  3920
CAAACTCTTTCATCTTCCCATTCGCTGTCTGTATTTCTCCATAACTATATCTTTCTTCCTCTGTCTCCGCCCACCCACATTTTTCTCTTTCCCTTTCTCTCTTTGGGGACCCTCCCCCATGCTCCCCTGCCCCATCCCCT  4060
```

Table V (Continued)

```
                                                              50                                           60                                            70
                                                pAsnTyrGlnLeuTrpLeuGlyArgHisAsnLeuPheAspAspGluAsnThrAlaGlnPheValHisValSerGluSerPheProHisProGly
TTTCCCCTTCCCGTCTTCTCATCCCTCCATTCCCATCTTTCCCCAGCAATTACCAGCTCTGGCTGGGTCGCCACAACTTGTTTGACGACGAAAACACAGCCCAGTTTGTTCATGTCAGTGAGAGCTTCCCACACCCTGGC   4200
  •      80           •            90                                         100                                              110                                            120
PheAsnMetSerLeuLeuGluAsnHisThrArgGlnAlaAspGluAspTyrSerHisAspLeuMetLeuLeuArgLeuThrGluProAlaAspThrIleThrAspAlaValLysValValGluLeuProThrGlnGluPr
TTCAACATGAGCCTCCTGGAGAACCACACCCGCCAAGCAGACGAGGACTACAGCCACGACCTCATGCTGCTCCGCCTGACAGAGCCTGCTGATACCATCACAGACGCTGTGAAGGTCGTGGAGTTGCCCACCCAGGAACC   4340
                  130                              140 •
oGluValGlySerThrCysLeuAlaSerGlyTrpGlySerIleGluProGluAsnP
CGAAGTGGGGAGCACCTGTTTGGCTTCCGGCTGGGGCAGCATCGAACCAGAGAATTGTATGTGGGGGCAGACTGTGTAGCCCAAGGCGGGGATGGGGACTCCTGCGTCCAAGGGAGAAAGGGCCAGGGAAACAGGTGAGG   4480
                                                     150                                        160                                     170
                                         heSerPheProAspAspLeuGlnCysValAspLeuLysIleLeuProAsnAspGluCysLysLysAlaHisValGlnLysValThrAspPheMetLeuCysValGl
TCGGGCTGCAGCCCTTTTTCTCCCGGGTTCGTAGTCTCATTTCCAGATGATCTCCAGTGTGTGGACCTCAAAATCCTGCCTAATGATGAGTGCAAAAAAGCCCACGTCCAGAAGGTGACAGACTTCATGCTGTGTGTCGG   4620
     180
vHisLeuGluGlyGlyLysAspThrCysVal
ACACCTGGAAGGTGGCAAAGACACCTGTGTGGTGAGGCAGCCCTGCCCCCAGGGTCTGGAAGGGCTGAGGGAGGGGACTCAGCCTCTGAACTGGCTTCTGAGAGCTAACCAGGCATCTGCTTCACTGCTTCCCAGCTAGC   4760

TGTAGCCACTCGCCCCATCAGTGCCCCAGCTCCCCCTCCTTCCCCGCCCATCCAGGGACAACTGCATCTCACCCCCCACACCAGAGTTCACCGTTCCTCGTGGTAATGTGTTGTTACCGTCGAGTCAGAGAGTAGTCCTG   4800

GAGAGGTGGCCTCTGCGATGTGCCCGCAGGGGCAGCGTCCTGCAGATGGTCCTGCCCCTCCTCCCCCTAACCTGTCTGCAGGCACTGTCCACCTGGACCCTGCCCCATGTGCAGGAGCTGGACCCTGAGGTCCCTTCCCC   5040

CTTGGCCAGGACTGGAGACCCTGTCCCCTCTGTGGGAATCCCTGCCCACCTTCCTCTGGGAGTGGGCACTGGAGGCCCTGTCTGCGAGCTGGGGCTCCCCAGGCAGAACTTGGGCCCCGTAGACCTTTCTCACTCTCCCC   5180
                     190                                         200                                        210                                        220
            GlyAspSerGlyGlyProLeuMetCysAspGlyValLeuGlnGlyValThrSerTrpGlyTyrValProCysGlyThrProAsnLysProSerValAlaValArgValLeuSerTyrValL
TCCCACCTTGTGCTCCCAGGGTGATTCAGGGGGCCCGCTGATGTGTGATGGTGTGCTCCAAGGTGTCACATCATGGGGCTACGTCCCTTGTGGCACCCCCAATAAGCCTTCTGTCGCCGTCAGAGTGCTGTCTTATGTGA   5320
   230                   238
ysTrpIleGluAspThrIleAlaGluAsnSerEnd
AGTGGATCGAGGACACCATAGCGGAGAACTCCTGAACGCCCAGCCCTGTCCCCTACCCCCAGTAAAATCAAATGTGCATCCAATGTGTGTCACGTTCTGCCATCACCTATCTTTCCAGATGTGGTGCACCTGGACCCACT   5460

CGGCTGAGGCTGGGGCCACCCCAGCTGTGTCAATCTCATGCCTGGAAGTCTGAGGTCACCAGCAGGTGGGGAAAGGAAGGGGCTGTGACAGGTGCAGACAAACAGGCAGGGAAAGGCTCAGATCCCAAAGGGCGAGTCAG   5600

GGACGCCAGAACAGTTCACTCACACTAGGATGGACCCTGCCCTGGTGGGGAGGTGGGGGGCAATGGAAGGTCTGAGGCTGAGAGGT                                                              5686
```

## Example 6

Table VI represents the nucleotide sequence of a manufactured DNA encoding the recombinant mature kallikrein polypeptide of the present invention. The manufactured DNA was constructed according to the methods described by Alton et al., PCT application WO 83/04053. This manufactured (synthetic) gene has codons preferred for E. coli expression. EcoRI and NdeI sites were added 5′ to the initiation codon ATG, and PstI and BamHI sites 3′ to the termination codon TAA. The resulting sequence is depicted in Table VI-A. The entire synthetic gene was cloned into pUC119 cut with EcoRI and BamHI and the resulting plasmid then sequenced. The gene was removed by digestion with NdeI and BamHI, then inserted into the E. coli expression vector pCFM1156 as described in commonly owned U.S. Ser. No. 004,379 hereby incorporated by reference. The resulting expression plasmid was used to transfect E. coli host FM5 as described in Burnette et al., BIO/TECHNOLOGY, Vol. 6, 699 (1988). The transformed E. coli was grown in brain/heart infusion (BHI) medium containing 20 µg/ml kanamycin at 28°C until $OD_{600}$ = 0.1, then shifted to 42°C for 4-6 hr for maximal expression. The level of kallikrein expression was approximately 25% of the total cellular protein as estimated from SDS - Polyacrylamide gel electrophoresis analysis of whole cell lysates.

The E. coli expressed kallikrein was extracted from inclusion bodies isolated from a bacterial cell paste by solubilizing in 8 M urea, pH 3.5 for 2 hr. The lysate was clarified by centrifugation at 5000 x g for 30 minutes. The clear lysate was diluted 10-fold and adjusted to pH 9-11 with sodium hydroxide. The solution was left stirring overnight at 4°C, then 2-mercaptoethanol added to a final concentration of 0, 50, or 100 mM. At the completion of oxidation, pH of the solution was adjusted to 8 with acetic acid and then reclarified as before.

The efficiency of refolding into an immunologically detectable molecule was determined by RIA as described in Example 7 to be approximately 0.42% of the proteins present in inclusion bodies.

**Table VI**

```
                                               30           NcoI                    60
ATG ATT GTA GGC GGT TGG GAA TGT GAA CAA CAT AGC CAG CCA TGG CAG GCT GCG CTG TAT
Met Ile Val Gly Gly Trp Glu Cys Glu Gln His Ser Gln Pro Trp Gln Ala Ala Leu Tyr

                                               90                                  120
CAC TTT TCT ACC TTT CAA TGC GGC GGT ATC CTG GTG CAC CGT CAG TGG GTT CTG ACC GCG
His Phe Ser Thr Phe Gln Cys Gly Gly Ile Leu Val His Arg Gln Trp Val Leu Thr Ala

                                              150                                  180
GCA CAC TGC ATC AGC GAT AAT TAT CAA CTG TGG CTC GGC CGC CAC AAC CTG TTC GAT GAC
Ala His Cys Ile Ser Asp Asn Tyr Gln Leu Trp Leu Gly Arg His Asn Leu Phe Asp Asp

                                              210                                  240
GAA AAC ACT GCA CAG TTC GTT CAC GTG AGC GAA TCC TTT CCG CAC CCG GGC TTC AAC ATG
Glu Asn Thr Ala Gln Phe Val His Val Ser Glu Ser Phe Pro His Pro Gly Phe Asn Met

         XhoI                                 270                                  300
TCT CTG CTC GAG AAT CAC ACC CGT CAG GCG GAT GAA GAC TAT AGC CAT GAC CTG ATG CTG
Ser Leu Leu Glu Asn His Thr Arg Gln Ala Asp Glu Asp Tyr Ser His Asp Leu Met Leu

                                              330                                  360
CTG CGT CTG ACC GAA CCG GCA GAT ACC ATC ACC GAT GCG GTT AAA GTG GTT GAA CTG CCG
Leu Arg Leu Thr Glu Pro Ala Asp Thr Ile Thr Asp Ala Val Lys Val Val Glu Leu Pro

                                              390                                  420
ACT CAG GAA CCG GAA GTG GGC TCC ACC TGT CTG GCG TCT GGT TGG GGC AGC ATC GAA CCG
Thr Gln Glu Pro Glu Val Gly Ser Thr Cys Leu Ala Ser Gly Trp Gly Ser Ile Glu Pro

                                              450                                  480
GAA AAC TTC AGC TTC CCG GAT GAC CTG CAA TGC GTG GAC CTG AAA ATT CTG CCG AAC GAC
Glu Asn Phe Ser Phe Pro Asp Asp Leu Gln Cys Val Asp Leu Lys Ile Leu Pro Asn Asp

                                           BstEII                                  540
GAA TGC GAA AAA GCG CAC GTG CAA AAG GTT ACC GAT TTC ATG CTG TGC GTG GGC CAT CTG
Glu Cys Glu Lys Ala His Val Gln Lys Val Thr Asp Phe Met Leu Cys Val Gly His Leu

                                              570                                  600
GAG GGT GGT AAA GAT ACG TGT GTG GGT GAT TCT GGC GGC CCG CTG ATG TGC GAC GGT GTT
Glu Gly Gly Lys Asp Thr Cys Val Gly Asp Ser Gly Gly Pro Leu Met Cys Asp Gly Val

                                              630                                  660
CTT CAG GGC GTT ACC AGC TGG GGT TAC GTT CCG TGT GGT ACC CCG AAC AAA CCG TCT GTG
Leu Gln Gly Val Thr Ser Trp Gly Tyr Val Pro Cys Gly Thr Pro Asn Lys Pro Ser Val

                                              690                                  720
GCG GTT CGT GTG CTG AGC TAC GTT AAA TGG ATC GAA GAT ACC ATT GCG GAG AAC AGC TAA
Ala Val Arg Val Leu Ser Tyr Val Lys Trp Ile Glu Asp Thr Ile Ale Glu Asn Ser End
```

Table VI-A

```
                                                                    EcoRI NdeI
                                                                      AATTCCAT
                                                                          GGTA
                                   30                  NcoI                 60
ATG ATT GTA GGC GGT TGG GAA TGT GAA CAA CAT AGC CAG CCA TGG CAG GCT GCG CTG TAT
TAC TAA CAT CCG CCA ACC CTT ACA CTT GTT GTA TCG GTC GGT ACC GTC CGA CGC GAC ATA

                                   90                                      120
CAC TTT TCT ACC TTT CAA TGC GGC GGT ATC CTG GTG CAC CGT CAG TGG GTT CTG ACC GCG
GTG AAA AGA TGG AAA GTT ACG CCG CCA TAG GAC CAC GTG GCA GTC ACC CAA GAC TGG CGC

                                  150                                      180
GCA CAC TGC ATC AGC GAT AAT TAT CAA CTG TGG CTC GGC CGC CAC AAC CTG TTC GAT GAC
CGT GTG ACG TAG TCG CTA TTA ATA GTT GAC ACC GAG CCG GCG GTG TTG GAC AAG CTA CTG

                                  210                                      240
GAA AAC ACT GCA CAG TTC GTT CAC GTG AGC GAA TCC TTT CCG CAC CCG GGC TTC AAC ATG
CTT TTG TGA CGT GTC AAG CAA GTG CAC TCG CTT AGG AAA GGC GTG GGC CCG AAG TTG TAC

         XhoI                     270                                      300
TCT CTG CTC GAG AAT CAC ACC CGT CAG GCG GAT GAA GAC TAT AGC CAT GAC CTG ATG CTG
AGA GAC GAG CTC TTA GTG TGG GCA GTC CGC CTA CTT CTG ATA TCG GTA CTG GAC TAC GAC

                                  330                                      360
CTG CGT CTG ACC GAA CCG GCA GAT ACC ATC ACC GAT CCG GTT AAA GTG GTT GAA CTG CCG
GAC GCA GAC TGG CTT GGC CGT CTA TGG TAG TGG CTA GGC CAA TTT CAC CAA CTT GAC GGC

                                  390                                      420
ACT CAG GAA CCG GAA GTG GGC TCC ACC TGT CTG GCG TCT GGT TGG GGC AGC ATC GAA CCG
TGA GTC CTT GGC CTT CAC CCG AGG TGG ACA GAC CGC AGA CCA ACC CCG TCG TAG CTT GGC

                                  450                                      480
GAA AAC TTC AGC TTC CCG GAT GAC CTG CAA TGC GTG GAC CTG AAA AAT CTG CCG AAC GAC
CTT TTG AAG TCG AAG GGC CTA CTG GAC GTT ACG CAC CTG GAC TTT TAA GAC GGC TTG CTG

                                 BstEII                                    540
GAA TGC GAA AAA GCG CAC GTG CAA AAG GTT ACC GAT TTC ATG CTG TGC GTG GGC CAT CTG
CTT ACG CTT TTT CGC GTG CAC GTT TTC CAA TGG CTA AAG TAC GAC ACG CAC CCG GTA GAC
                                  510

                                  570                                      600
GAG GGT GGT AAA GAT ACG TGT GTG GGT GAT TCT GGC GGC CCG CTG ATG TGC GAC GGT GTT
CTC CCA CCA TTT CTA TGC ACA CAC CCA CTA AGA CCG CCG GGC GAC TAC ACG CTG CCA CAA

                                  630                                      660
CTT CAG GGC GTT ACC AGC TGG GGT TAC GTT CCG TGT GGT ACC CCG AAC AAA CCG TCT GTG
GAA GTC CCG CAA TGG TCG ACC CCA ATG CAA GGC ACA CCA TGG GGC TTG TTT GCC AGA CAC

                                  690                                      720
GCG GTT CGT GTG CTG AGC TAC GTT AAA TGG ATC GAA GAT ACC ATT GCG GAG AAC AGC TAA
CGC CAA GCA CAC GAC TCG ATG CAA TTT ACC TAG CTT CTA TGG TAA CGC CTC TTG TCG ATT

Pst I
CTGCAG
GACGTCCTAG
      BamHI
```

Example 7

Radioimmunoassay for Kallikrein

The radioimmunoassay procedure employed for quantitative detection of kallikrein was a modification of the procedure described by Shimamoto et al., [J. Clin. Endocrinol & Met. 51: 840-848 (1980)]:

The assay buffer employed was phosphate-buffered saline (PBS; 0.14 M NaCl in 0.01 M $Na_2HOP_4$-$NaH_2PO_4$, pH 7.0) containing 0.1% BSA. The assay utilizes the following reagents and samples: 200 µl aliquots of samples or purified human urinary kallikrein (24 pg - 6250 pg) standards containing appropriate dilutions in the assay buffer; 100 µl of rabbit antiserum raised against purified human urinary kallikrein at 1:250,000 dilution; 100 µl of $^{125}$I-human urinary kallikrein (Sp. Act. ~1.5 x $10^8$ cpm per µg kallikrein) 50,000 cpm. All samples and standards were assayed in duplicate. Assay tubes were incubated at 37°C for 2 hours then at 4°C overnight. Antibody bound kallikrein was separated from free kallikrein by adding a formalin fixed staphylococcus aureus (Cowan Strain) cell suspension, IgGsorb (Enzyme Center, Inc). To each tube, fifty µl of 10% IgGsorb was added and let the reaction mixture stand at room temperature for 30 min. The tubes were centrifuged and the resulting pellet was washed twice with a wash buffer comprising 0.05 M Tris-HCl, (pH 8.9), 2% BSA, 0.1% SDS and 0.1% Triton X-100 and counted in a gamma counter. The assay detects kallikrein in a range from about 24 pg to about 6250 pg. The kallikrein content of an unknown sample was determined by comparison to a standard containing a known quantity of pure human urinary kallikrein.

Example 8

Human Grandular Kallikrein Gene Sequences

Nucleotide sequence analyses of the two independent positive human genomic kallikrein clones designated as λHK65a and λHK76a were carried out and results obtained for the kallikrein gene containing region of clone λHK65a are set out in Table V. Both clones have identical kallikrein protein coding sequences and identical intron sequences that were completed and the restriction endonuclease map of the human kallikrein gene from clone λHK65a is shown in Table VII.

The protein coding region of the gene is divided by four intervening sequences or introns. Since the transcription initiation site of the mRNA for kallikrein has not been determined due to lack of human tissue mRNA, the boundary on the 5′ side of exon I is undefined. The exons were identified by comparison of the nucleotide sequence to the amino acid sequence of human urinary kallikrein shown in Table I and by comparison with the cDNA sequence published [Baker et al., supra, and Fukushima et al., supra]. The exon-intron boundaries of the kallikrein gene conform to consensus splice rules, [Mount, Nucleic Acids Res. 10: 459-472 (1982)]. In Table IV, the initial sequence appears to comprise the 5′ end untranslated region (802 bp) of the gene which may contain enhancer/promoter like functions,

Table VII

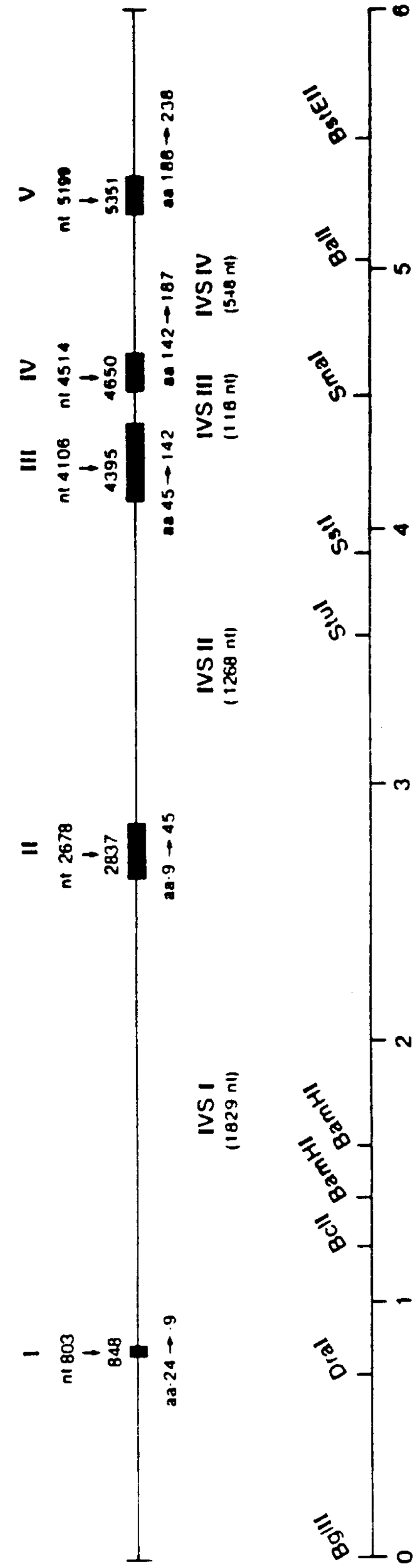


that leads up to a translated DNA region, the first exon, coding for the first 15 amino acids (-24 through -10, and part of residue -9). Then follows an intervening sequence (IVS) of 1829 base pairs. The second exon immediately followed codes for amino acid residues glycine -9 through aspartic acid 45. The second IVS that follows comprises 1268 base pairs. The third exon codes for amino acid residues aspartic acid 45 through phenylalanine 142, the third IVS of 118 base pairs. The fourth exon encodes amino acid residues phenylalanine -142 through valine 187, the fourth IVS of 548 base pairs. The last exon codes for amino acid residues Glycine 188 through serine 238 and a stop codon (TGA).

There is a 46 bp untranslated region at the 3′ end of the last exon. The nucleotide sequence 15 bp upstream from the site contain a sequence AGTAAA resembling the consensus polyadenylation signal sequence AATAAA and the related sequences normally found at this location. [Nevins, Annu. Rev. Biochem. 52, 441-466 (1983)].

The kallikrein gene encodes a protein of 262 amino acids. Based on the $NH_2$-terminal amino acid sequence of purified human urinary kallikrein, the last 238 residues correspond to the mature active protein with a calculated $M_r$ of 26403 in an unglycosylated form. The sequence of the first 17 amino acids, predominantly hydrophobic residues, is consistent with this region encoding a signal peptide, [Watson, Nucleic Acids Res. 12: 5145-5164 (1984)] and the following 7 amino acid residues being the activation peptide or propeptide, [Takahashi et al., J. Biochem 99, 989-992 (1986)].

The amino acid sequence starting at position -7 through -1 corresponds to the propeptide sequence in the recombinant prokallikrein and that starting at +1 corresponds to the sequence of the amino terminus of expressed recombinant mature kallikrein product of the present invention in CHO cells. As indicated in Table V, the mature protein has three potential sites for Asn-linked glycosylation, amino acid residues 78, 84 and 141 in the third exon of the gene, according to the rule of Asn-Xaa-Ser/Thr, [Marshall, Biochem. Soc. Symp. 40, 17-26 (1974)], and has 10 cysteine residues.

Example 9

Table VIII below illustrates the extent of polypeptide sequence homology between human and monkey kallikrein of the present invention. In the upper continuous line of the Table, three letter designations for amino acids are employed to represent the deduced translated polypeptide sequences of human kallikrein of the present invention commencing with residue -24 and the amino acid residues appearing in the lower continuous line shows the differences in the deduced polypeptide sequence of monkey kallikrein also commencing at assigned residue number -24. Dashes are employed to highlight missing amino acid residues in the monkey kallikrein sequence that are present in human kallikrein of the present invention.

Table VIII

**Comparison of Amino Acid Sequences of Human and Monkey Kallikrein**

| | | | | | | | | | | | | | | | | | | | |
|---|---|---|---|---|---|---|---|---|---|---|---|---|---|---|---|---|---|---|---|
| | | | | | | | | | | | | | | | | -24 | | | |
| | | | | | | | | | | | | | | | | Met | Trp | Phe | Leu |
| -20 | | | | | | | | | | -10 | | | | | | | | | -1 |
| Val | Leu | Cys | Leu | Ala | Leu | Ser | Leu | Gly | Gly | Thr | Gly | Ala | Ala | Pro | Pro | Ile | Gln | Ser | Arg |
| | | | | | | | | | | | | | Arg | | | | | | |
| +1 | | | | | | | | | 10 | | | | | | | | | | 20 |
| Ile | Val | Gly | Gly | Trp | Glu | Cys | Glu | Gln | His | Ser | Gln | Pro | Trp | Gln | Ala | Ala | Leu | Tyr | His |
| | | | | | | | — | — | — | | | | | | | | | | |
| | | | | | | | | | 30 | | | | | | | | | | 40 |
| Phe | Ser | Thr | Phe | Gln | Cys | Gly | Gly | Ile | Leu | Val | His | Arg | Gln | Trp | Val | Leu | Thr | Ala | Ala |
| | | | | | | | | | | | | Pro | | | | | | | |
| | | | | | | | | | 50 | | | | | | | | | | 60 |
| His | Cys | Ile | Ser | Asp | Asn | Tyr | Gln | Leu | Trp | Leu | Gly | Arg | His | Asn | Leu | Phe | Asp | Asp | Glu |
| | | | | | | | | | 70 | | | | | | | | | | 80 |
| Asn | Thr | Ala | Gln | Phe | Val | His | Val | Ser | Glu | Ser | Phe | Pro | His | Pro | Gly | Phe | Asn | Met | Ser |
| Asp | | | | | | | | | | | | | | | | | | | |
| | | | | | | | | | 90 | | | | | | | | | | 100 |
| Leu | Leu | Glu | Asn | His | Thr | Arg | Gln | Ala | Asp | Glu | Asp | Tyr | Ser | His | Asp | Leu | Met | Leu | Leu |
| | | Lys | | | | | | | | — | | | | | | | | | |
| | | | | | | | | | 110 | | | | | | | | | | 120 |
| Arg | Leu | Thr | Glu | Pro | Ala | Asp | Thr | Ile | Thr | Asp | Ala | Val | Lys | Val | Val | Glu | Leu | Pro | Thr |
| | | | Gln | | | Glu | — | | | | | | Gln | | | | | | |
| | | | | | | | | | 130 | | | | | | | | | | 140 |
| Gln | Glu | Pro | Glu | Val | Gly | Ser | Thr | Cys | Leu | Ala | Ser | Gly | Trp | Gly | Ser | Ile | Glu | Pro | Glu |
| | | | | | | | | | 150 | | | | | | | | | | 160 |
| Asn | Phe | Ser | Phe | Pro | Asp | Asp | Leu | Gln | Cys | Val | Asp | Leu | Lys | Ile | Leu | Pro | Asn | Asp | Glu |
| | | | | | | | | | | | | | Glu | | | | | | |
| | | | | | | | | | 170 | | | | | | | | | | 180 |
| Cys | Lys | Lys | Ala | His | Val | Gln | Lys | Val | Thr | Asp | Phe | Met | Leu | Cys | Val | Gly | His | Leu | Glu |
| | Ala | | | | Thr | | | | | Glu | | | | | Ala | | | | |
| | | | | | | | | | 190 | | | | | | | | | | 200 |
| Gly | Gly | Lys | Asp | Thr | Cys | Val | Gly | Asp | Ser | Gly | Gly | Pro | Leu | Met | Cys | Asp | Gly | Val | Leu |
| | | | | | | | | | | | | | | Thr | | | | | |
| | | | | | | | | | 210 | | | | | | | | | | 220 |
| Gln | Gly | Val | Thr | Ser | Trp | Gly | Tyr | Val | Pro | Cys | Gly | Thr | Pro | Asn | Lys | Pro | Ser | Val | Ala |
| | | | | | | | | Ile | | | | Ser | | | | | Ala | | Phe |
| | | | | | | | | | 230 | | | | | | | | 238 | | |
| Val | Arg | Val | Leu | Ser | Tyr | Val | Lys | Trp | Ile | Glu | Asp | Thr | Ile | Ala | Glu | Asn | Ser | | |
| | Lys | | | | | | | | | | | | | | | | | | |

Example 10

Construction of the Plasmid pDSHK1 for the Expression of Human Kallikrein Gene in Mammalian Cells

For the expression of the human kallikrein gene, a 7 kb Bgl II-XbaI fragment from lambda clone λHK65a, that contains the entire kallikrein gene, was first isolated and then inserted into BamHI/XbaI doubly-digested plasmid vector pUC118. The resulting pUC118-based kallikrein clone was subjected to the Henikoff deletion procedures [Henikoff, Gene 28: 351-359 (1984)] to generate clones containing the human kallikrein gene with its 3′-flanking sequences deleted to various extent. In particular, the plasmid was opened by digesting with restriction enzymes SphI and SalI. The combination of ExoIII nuclease and SI nuclease was utilized to digest the insert from its 3′ end (the XbaI site) for various time intervals. Thereafter, the plasmid DNA was treated with DNA polymerase large fragment (Klenow enzyme) to fill the ends for subsequent blunt-end ligation with $T_4$ DNA ligase. The recircularized plasmid DNA was used to transform the DH5$\alpha$ E. coli host, (Bethesda Research Laboratories, Cat. No. 8263SA). The resulting transformants were analyzed by sizing the inserts and by DNA sequencing. One of the deletion clones, pHK102, contains the entire human kallikrein gene insert with 801 bp upstream from the protein initiation codon and 232 bp downstream from the termination codon. The pHK102 contains a DraI site 67 bp upstream from the protein initiation codon of the kallikrein gene and a HindIII site which was carried over from the pUC118 237 bp downstream from the termination codon. The pHK102 DNA was digested with DraI and HindIII and the approximate 4.85 Kb DraI-HindIII fragment was isolated and briefly digested with Bal31-Slow nuclease. The resulting DNA fragment was ligated to the BamHI cleaved expression vector pDSVL (pDSVL contains a murine dihydrofolate reductase gene and SV40 late promoter as described in PCT application No. WO 85/02610), that have been end-filled with DNA polymerase large fragment (Klenow enzyme). The kallikrein expression plasmid thus obtained was designated as pDSHK1, which contains the entire human kallikrein gene including 64 bp 5′ to the initiation codon and 232 bp 3′ from the termination codon. The detailed construct of pDSHK1 is depicted in Figure 1. The pDSHK1 plasmid contains a murine DHFR minigene as a EcoRI-PstI fragment from pMgI; SV40 origin of replication and early/late promoters in the HindIII-BamHI fragment; SV40 nt 2538-2770 in the BamHI-BclI fragment; pBR322 nt 2448-4362 in the HindIII-EcoRI fragment; and the 4.85 kb DraI-HindIII fragment of the human kallikrein gene. The SV40 late promoter is used to drive the expression of the human kallikrein gene. This pDSHK1 plasmid was used to transfect African green monkey kidney cells COS-1 for transient expression or transfect $DHFR^-$ CHO cells for stable expression.

Example 11

A. Transient Expression of Human Kallikrein Gene in African Green Monkey Kidney Cells, COS-1 Cells

A calcium phosphate microprecipitate of covalent circular DNA, pDSHK1, at 1.5 $\mu$g DNA plus 10 $\mu$g mouse liver DNA as carrier per 4 x $10^5$ cells was used to transfect COS-1 cells. The cells were grown in high glucose DMEM medium supplemented with 10% fetal bovine serum plus penicillium, streptomycin and glutamine. A three-day conditioned medium was determined to contain 5.1 ng immunoreactive kallikrein per ml using the radioimmunoassay procedure of Example 7.

B. Expression of Human Kallikrein Gene in Chinese Hamster Ovary (CHO) Cells with Plasmid pDSHK1

(1) Transfection of CHO Cells With the Circular Plasmid DNA of pDSHK1

Chinese hamster ovary $DHFR^-$ cells (CHO $DHFR^-$ cells) [Urlaub et al., (1980) Proc. Nat. Acad. Sci. (U.S.A.), 77, 4461] lack the enzyme dihydrofolate reductase (DHFR) due to mutations in the structural genes and therefore require the presence of glycine, hypoxanthine, and thymidine in the culture media for growth. The cells were grown as a monolayer in medium C comprising Dulbecco's modified Eagle's medium (DMEM) supplemented with 5% fetal bovine serum, 1% nonessential amino acids, 1% hypoxanthine and thymidine, and penicillin, streptomycin, and glutamine. One day prior to transfection, cells were plated at the approximate density of 3 x $10^6$ cells per 10 cm culture dish. A calcium phosphate microprecipitate procedure by a modification of the methods of Graham et al., [Virology 52: 456-467 (1973)], and Wigler et al., [Cell 11: 223-232 (1977)] was employed to introduce pDSHK1 DNA with a final concentration of 6 to 7 $\mu$g of plasmid DNA per $10^6$ cells. Cells that had been transformed with and were expressing the DHFR gene (and thereby the kallikrein gene) survived and proliferated in the selective medium comprising DMEM supplemented with 10% dialyzed fetal bovine serum, 1% nonessential amino acids, glutamine, penicillin, and streptomycin, but no hypoxanthine and thymidine. The cells that grew in the selective medium were considered as stable transformants. The culture conditioned medium of the stable transformants was collected and assayed for the immunoreactive human kallikrein by the radioimmunoassay procedure, described in Example 7. The results indicated that the immunologically reactive recombinant human kallikrein products were secreted at the level of 6 ng/ml from a 3-day conditioned medium.

(2) Transfection of CHO cells with linear DNA fragment of pDSHK1

Plasmid pDSHK1 (9.85 kb) was digested by restriction enzymes EcoRI and HindIII. The resulting digest was applied to a 0.7% agarose gel electrophoresis to separate the kallikrein insert containing fragment from the prokaryotic DNA fragment which originated from pBR plasmid. The 7.9 Kb DNA fragment, that contains the DHFR gene, the SV40 regulatory sequences, and the kallikrein gene, was isolated from the agarose gel. The transfection of CHO cells with this linear DNA fragment was carried out in 60 mm culture dishes at the cell density of 1 x $10^6$ per dish with 10 $\mu$g of 7.9 Kb DNA fragment added per dish as previously described in Example 11B(1) for the closed circular DNA. Seventeen hours after adding the DNA to the cells, the medium was aspirated and replaced with a fresh medium. Three days later, the cells were trypsinized from two of 60 mm dishes and transferred to two 75-$cm^2$ culture flasks. On the following day, the medium was replaced with the selective medium. From this point on, the cells were maintained and subcultured in the selective medium for approximately four weeks and were designated as stable transformants. The stable transformants have a secretion level of 0.27 $\mu$g/ml of conditioned medium obtained from a 14-day culture in serum-free condition, Ham's F-12/DME 1:1 mixture supplemented with glutamine.

C. Amplification of the Cloned Human Kallikrein Gene (HK) in CHO $DHFR^-$ Cells Containing pDSHK1

The quantity of recombinant kallikrein produced by the stable transformants may be increased by gene amplification with methotrexate treatment to yield new cell strains having higher productivity. To amplify the cloned kallikrein gene, the stable transformants were subjected to a series of increasing concentration of methotrexate treatment. Initially, the cells were shifted from the selective medium to a methotrexate medium. The methotrexate medium is composed of DMEM supplemented with 5% fetal calf serum, 1% nonessential amino acids, glutamine, penicillin and streptomycin, plus methotrexate at desired concentration.

Cell strain CHO-DSHK1-C1 (stable transformants that originate from circular DNA transfected cells) was subjected to treatments with increasing methotrexate concentrations (0 nM, 20 nM, 60 nM, 300 nM, and 1 $\mu$M) to select out the higher producing cell strains. After about four weeks of culturing in a given methotrexate-containing media, the near confluent cells were then fed with serum-free media without methotrexate. Representative 7-day medium samples from serum-free culture (Ham's F-12/DMEM 1:1 mixture) from each amplification step were assayed using the radioimmunoassay procedure of Example 7 and determined to contain 10, 42, 110, 693 and 708 ng human kallikrein/ml, respectively. On the other hand, the cell strain CHO-DSHK1-L1 (stable transformants that originate from linear DNA transfected CHO cells) was also subjected to amplification with increasing concentrations of methotrexate (0 nM, 30nM, 60 nM, 300 nM, 1 $\mu$M and 5 $\mu$M). The kallikrein content in the conditioned media was determined using the radioimmunoassay (RIA) procedure described in Example 7. The results based on 7-day samples are shown in Table IX.

Table IX

Expression of Kallikrein by CHO-DSHK1-L1 Cells Different Stages of Amplification with Methotrexate

| Methotrexate Concentration | Culture Media | Days of Conditioned Media | Kallikrein Concentration (µg/ml) |
|---|---|---|---|
| 0 | SF[1] | 14 | 0.27 |
| 60 nM | SF | 7 | 1.44 |
| | SF+PC-1[2] | 7 | 1.92 |
| 300 nM | SF+PC-1 | 7 | 2.34 |
| | SF+PC-1+Gln[3] | 7 | 3.57 |
| | SF+PC-1[4] | 7 | 4.06 |
| 1 µM | SF+PC-1 | 7 | 1.93 |
| | | 10 | 2.80 |
| | | 12 | 4.87 |
| | 5% serum | 7 | 1.74 |
| 5 µM | 5% serum | 7 | 0.82 |

1. SF = serum-free medium, i.e. Ham's F12/DMEM 1:1 mixture.
2. PC-1 = a supplement for serum-free culture, obtained from Ventrex (Portland, Maine).
3. Glutamine concentration is 8 mM instead of the regular 2 mM.
4. The cells used in this experiment had been previously maintained for 7 days in PC-1 supplemented serum-free medium, and subsequently 3 days in methotrexate medium.

The highest expression level was 4.06 µg human kallikrein/ml for a 7-day conditioned medium, observed with 300 nM methotrexate treated cells that have been transfected with linearized kallikrein DNA.

D. Expression of Human Kallikrein Gene in CHO cells with Plasmid pDGHK-L1A

(1) Construction of pDGHK-L1A Expression Vector

pDGHK-L1A as depicted in Figure 3, consists of the following: (i) pBR322 nt 2448-4362 as a HindIII-EcoRI fragment; (ii) a DHFR minigene as an EcoRI-PstI fragment from pMgl with a deletion in the 3′ untranslated region made by removing a BglII-BglII fragment of 556 bp, and then end filled with klenow enzyme; (iii) A bidirectional SV40 termination sequence of 237 bp originally as a BamHI-BclI fragment (SV40 nt 2770-2553), adding synthetic linker-1 to BclI (destroying the BclI site and generating a PstI site), and adding synthetic linker-2 to BamHI end of the fragment to create a SalI site; (iv) The kallikrein gene (nt 801- nt 5370 in Table V) with synthetic linker-3 added to DraIII site at nt 801 and with a SalI site was created by insertion of GA using site-directed mutagenesis between nt 15 and nt 16 past termination codon TGA, generating the sequence TGAACGCCCAGCCCTGTA(GA)C; (v) A rat glucose-regulated protein (GRP78) gene promoter which contains SmaI-BsshII fragments (GRP78 nt-722 to nt-37) [Shin C. Chang et al., Proc. Natl. Acad. Sci. 84:680-684 (1987)]; and (vi) synthetic linker-4 to reinstate the sequence including the mRNA cap site and also to create a BamHI cloning site. The GRP promoter-kallikrein gene fusion was made by ligating the BamHI site of linker-4 to the BglII site of linker-4 to the BglII site of linker-3. Linker-5 was added to the SmaI end of the GRP promoter sequence and it was joined to the pBR322 derived sequence at HindIII.

Linker 1:

```
    GCAATGGCAACAACGTTGCCCCG
ACGTCGTTACCGTTGTTGCAACGGGCCTAG
PstI                     (BclI)
```

Linker 2:

```
SalI  BamHI
TCGACTG
    GACCTAG
```

Linker 3:

```
BglII      (DraIII)
GATCTCAAACAGACAACAT
    AGTTTGTCTGTT
```

Linker 4:

```
CGCGCTCGATACTGGCTGTGACTACACTGACTTGGACACTTGGCCTTTTGCGGGTTTGAG
    GAGCTATGACCGACACTGATGTGACTGAACCTGTGAACCGGAAAACGCCCAAACTCCTAG
BsshII                                                      BamHI
```

Linker 5:

```
Hind III    EcoRI          SmaI
AGCTTGAGTCCTGAATTCGAGCTCGGTACCC
    ACTCAGGACTTAAGCTCGAGCCATGGG
```

(2) Transfection of CHO Cells with Plasmid pDGHK-L1A:

CHO $D^-$ cells were grown in high glucose, high glutamine DMEM (Gibco Laboratories, Cat. #320-1965) supplemented with 10% fetal bovine serum (FBS), 0.1 mM non-essential amino acids, 13.6 μg/ml hypoxanthine, 7.6 μg/ml thymidine, and 2 mM glutamine. The 60 mm plates were seeded at a density of approximately 3.3 x $10^5$ cells/dish containing 5 ml of the medium and allowed to grow for about 20 hours at 37°C, 5% $CO_2$. The media from each plate was aspirated, fresh media added and cells allowed to grow an additional five hours at 37°C, 5% $CO_2$. The media was again aspirated and the cells washed once with PBS, then once with transfection buffer before cells were transfected with the linearized plasmid DNA, pDGHK-L1A. The transfection procedure was essentially the same as described by Bond and Wold (Bond, V.C. and Wold, B., Mol. & Cell. Biol. 7:2286-2293, 1987). The transfection buffer consisted of 5 mM NaCl, 120 mM KCl, 1.5 mM $Na_2HPO_4$ and 25 mM Tris, pH 7.5; then adding $CaCl_2$ to 1.4 mM and $MgCl_2$ to 0.5 mM. After mixing, the buffer was sterilized by filtering through a 0.22 μm Costar Microstar filter. Plasmid pDGHK-LIA, linearized with restriction enzyme PvuI was extracted once with phenol-chloroform, 1:1, saturated with 0.05 M Tris, 0.02 M EDTA, 0.01 M 2-mercaptoethanol, pH 8.0, then once with chloroform only. The extracted plasmid digest was precipitated with 1/2 volume 7.5 M ammonium acetate and 2 1/2 volumes absolute ethanol at -20 °C overnight. Precipitated plasmid DNA was redissolved in the sterile transfection buffer to give a final concentration of approximately 2.5 µg/ml and 5 µg/ml. Poly-L-ornithine (Sigma, Cat. #P4638), 200 mg/ml in 0.01 M Tris pH 7.5 sterilized through a 0.22 µm Costar Microstar filter, was added to 17 µg/ml and a plasmid DNA concentration of 2.5 µg/ml transfection mixture, and was added to 25 µg/ml and a plasmid DNA concentration of 5 µg/ml plasmid DNA transfection mixture . Transfection mixtures (0.5 ml/60 mm dish) were carefully layered onto cells containing dishes and incubated for 1 hour at room temperature in a laminar flow hood. At the end of the transfection treatment, 5 ml of culture media were added to each dish, which was then returned to 37 °C, 5% $CO_2$. The following day, i.e., approximately 16 hours later, the culture dishes were aspirated and fresh media added, returning the dishes to 37 °C, 5% $CO_2$ for 48 hours. Cells were trypsinized and replated to four 100-mm dishes (10 ml medium each) for each 60 mm dish. One day later, the media was aspirated and the cells were washed twice with PBS. Then the selective media (it contains high glucose, high glutamine DMEM supplemented with 10% dialyzed FBS, 2 mM glutamine, 0.1 mM non-essential amino acids) was added. Selective media was changed approximately every 3 days until 15 days post transfection, when individual colonies were visible. Colonies were tabulated, trypsinized and transferred to 25-$cm^2$ culture flasks, at a density of approximately 50 pooled colonies/flask. From 1 µg of transfected DNA, about 40-50 colonies were obtained.

(3) Amplification of the Cloned HK Gene in CHO DHFR-Cells Containing pDGHK-L1A

Amplification procedure for CHO $DHFR^-$cells containing linearized pDGHK-L1A was essentially the same as that described in Example 11C with the following modifications.

Cell strain CHO-DGHK-L1A-8 (Stable transformants that originate from pDGHK-L1A transfected cells) was subjected to two different schemes of treatment with methotrexate (MTX). One scheme of MTX concentration was:

0→20nM→60nM→200nM→600nM MTX

and the other was:

0→30nM→100nM→300nM→900nM.

The kallikrein content as secreted by the cells into the media was determined using RIA as described in Example 7. 60nM MTX and 100nM MTX amplified cells secreted the highest level of kallikrein, 1350ng/ml/day and 1725 ng/ml/day respectively. The results are shown in Table X below.

TABLE X

| The Expression Level of Kallikrein by CHO-DGHKL1A-8 Cells at Different Stages of Amplification with Methotrexate | |
|---|---|
| MTX (nM) | Daily Yield of rHK (ng/ml) |
| 0 | 630 |
| 30 | 561 |
| 60 | 1350 |
| 100 | 1725 |
| 200 | 191 |
| 300 | 61 |
| 600 | 167 |

Data was obtained from 175-$cm^2$ culture flasks containing 40 ml medium and approximately 1.5 x $10^7$ cells per flask. The medium used was DMEM/F12 1:1 containing PC-1 (10ml/500 ml medium), 6mM Glutamine and 0.1 mM non-essential amino acids.

Example 12

Isolation of Recombinant Human Kallikrein

Cell free culture media containing recombinant human kallikrein produced by CHO cells was pooled and concentrated using a diafiltrator having a 10,000 dalton molecular weight cutoff membrane filter. The concentrated sample was buffer exchanged with 10 volumes of 10 mM Tris-HCl buffer (pH 7.8). The crude recombinant kallikrein was added to a QA-sepharose column packed and equilibrated with 10 mM Tris-HCl buffer (pH 7.8). The column was washed with the equilibration buffer and the recombinant kallikrein was eluted with a linear gradient of from 0 to 0.5 M NaCl in the same buffer. Both active kallikrein and enzymatically inactive prokallikrein were eluted at fractions containing 0.3 to 0.4 M NaCl. The recombinant kallikrein and prokallikrein fractions were pooled and dialyzed against 10 mM Tris-HCl (pH 7.8) buffer to remove excessive sodium chloride. The dialysis proceeded for 2-3 days at 4°C, at which time prokallikrein was completely activated by kallikrein to generate mature kallikrein. The dialyzed activated kallikrein pool was added to a benzamidine-sepharose affinity column which was pre-equilibrated with 10 mM Tris-HCl (pH 7.8). The column washed with the equilibration buffer and kallikrein was eluted with the same buffer containing 2 M guanidine HCl The kallikrein fractions were pooled then precipitated with 70% saturated ammonium sulfate in 10 mM Tris-HCl (pH 7.8). The ammonium sulfate precipitated kallikrein fractions were dissolved in 10 mM Tris-HCl buffer (pH 7.8) and the dissolved sample was applied to a sephacryl S-300 gel filtration column which is equilibrated with the same buffer. The kallikrein fractions were eluted at molecular weights ranging from 30,000 to 45,000. The pooled kallikrein fractions contained highly purified active recombinant human mature kallikrein. A $C_4$ HPLC column (Vydac $C_4$ column 25 cm x 4.6 mm) chromatography was used to check the purity of the final product. The buffer gradient conditions used were as follows:

| Gradient: | | |
|---|---|---|
| t(min) | %A | %B |
| O | 75 | 25 |
| 60 | 30 | 70 |
| 70 | 30 | 70 |
| 71 | 75 | 25 |
| A = 0.1% TFA (Trifluoroacetic acid) | | |
| B = 90% $CH_3CN$/0.1%TFA ($CH_3CN$ = Acetonitrile) | | |
| Flow = 1 ml/min | | |

The results are shown below in Table XA:

TABLE XA

Isolation of Recombinant Human Tissue Kallikrein (TCHK009) Derived from CHO Cells

| Steps | Volumes (ml) | Protein ($OD_{280}$) | Enzyme ** Activity (units) | Specific Activity (U/OD) | RIA (mg) | Yield (%) | Purification fold | Comment |
|---|---|---|---|---|---|---|---|---|
| Culture Media | 16,000 | - | 4,400 | - | (40 mg) | - | - | |
| Diafiltration | 1,500 | 3,720 | 4,610 | 1.24 | (40 mg) | 100 | 1 | |
| QA-Sepharose | 200 | 432 | 6,750 | 15.63 | | - | 8.6 | |
| Dialysis-Autoactivation | 220 | 432 | 54,400 | 125.93 | 44 | 110 | - | |
| Benzamidine-Sepharose | | | | | | | | |
| Frx A *** | 40 | 57 | 20,000 | 350.87 | - | 72 | 33 | |
| Frx B | 24 | 55 | 18,130 | 329.64 | | | | |
| Sephacryl S-300 | | | | | | | | |
| Frx A | 37 | 27 | 14385 | 532.78 | 15. | 69 | 84 | 30% proform 70% active form |
| Frx B | 33 | 17 | 13407 | 788.65 | 11.3 | | | 7% proform 93% active |

* HUK concentration of 1 mg/ml has an OD = 1.6 at 280 nm. Specific activity = 1100 U/mg assayed by AcPheArgOet coupling enzyme procedure.

** Kallikrein activity was assayed by protease activity using AcPheArgOet as a substitute (Fiedler et al., Methods in Enzymology 80: 493-532, 1980).

*** Frx=Fraction

The resulting purified kallikrein was enzymatically active as determined using the following synthetic peptide: Ac-Phe-Arg-OEt-Nitroanilide as a substrate for an esterase assay according to the procedures described by Geiger et al., [Adv. Biosci. 17, 127 (1979)].

From different batches of culture media both kallikrein (the mature, active form) and prokallikrein (the inactive form) have been purified using similar purification procedures. The purified proenzyme was not enzymatically active. Active kallikrein was isolated from prokallikrein after autoactivation or after removal of the activation peptide in prokallikrein by incubation with trypsin.

Example 13

Sequence Analysis of Recombinant Kallikrein and Prokallikrein

Sequence determination of the purified recombinant kallikrein revealed that the purified protein is homogeneous and contains a single amino terminus. The partial N-terminal sequence is determined as:

```
    1   2   3   4   5   6   7   8   9   10  11  12  13
NH-Ile-Val-Gly-Gly-XXX-Glu-XXX-Glu-Gln-His-Ser-Gln-Pro-

14  15  16
XXX-Gln-Ala----------,
```

wherein XXX denotes residues that cannot be positively assigned. The result indicated that the N-terminal sequence of recombinant kallikrein is identical to the previously determined N-terminal sequence of human urinary kallikrein.

N-terminal sequence analysis of recombinant prokallikrein revealed that the proform contains a heptapeptide leader followed by the amino acid sequence of mature kallikrein, i.e.,

```
1   2   3   4   5   6   7   8   9   10  11  12  13
Ala-Pro-Pro-Ile-Gln-Ser-Arg-Ile-Val-Gly-Gly-XXX-Glu-

14  15  16  17  18  19  20
XXX-Glu-Gln-His-Ser-Gln-Pro----------,
```

where XXX denotes residues that cannot be positively assigned. This proform N-terminal sequence is also identical to the N-terminal sequence reported by Takahashi et al., supra.

Example 14

Kinongenase Assay for Kallikrein

The procedure used for measuring the kinin-generating activity of the recombinant kallikrein polypeptide of the present invention was essentially the same as the described by Shimamoto et al., Jap. Circ. J. 43: 147-152), (1979):

Purified human urinary kallikrein standards or recombinant kallikrein samples diluted (20 µl or 40 µl) in 0.1 M sodium phosphate (pH 8.5)/30 mM $Na_2$EDTA/3 mM phenanthroline containing 3 µg of purified bovine low molecular weight kininogen in a total volume of 0.5 ml were incubated at 37 °C for 30 min. The reaction was terminated by boiling for 10 min. and 50 µl aliquots in duplicate were used to measure the amount of kinin-released by a kinin RIA with a rabbit antiserum against kinin. The kininogenase activity of recombinant kallikrein is expressed as the amount of generated kinin in µg/30 min/mg kallikrein.

Example 15

In Vivo Assay for Blood Pressure-Lowering Effect of Kallikrein

A. Rats

Male species of spontaneous hypertensive rats, about 250 grams body weight, were used in the study. Rats were anesthetized with sodium pentobarbital (50mg/Kg body weight) intraperitoneally. The common carotid artery was cannulated and then connected to a Statham pressure transducer. The right jugular vein was cannulated for administering kallikrein and changes in blood pressure were measured with a polygraph. Upon administration of the recombinant mature kallikrein of the present invention, lowering blood pressure levels in the hypertensive rats was observed.

B. Pigs

In order to carry out conscious animal experiments for the drug test the following protocol was used. Normal farm pigs weighing approximately 50kg, were sedated with 25 mg ketamine HCl, per Kg, IM, and anesthesia was induced with 20 mg sodium thyamylol per Kg, IV and was maintained with 1-2% halothane. A left thoracotomy was performed at the fourth or fifth rib space. Both an aortic and left atrial catheter were implanted and exteriorized through the back.

The animals were allowed to recover one week before undergoing an experiment. During the recovery period each animal was trained for the drug testing procedure. All animals were monitored daily to insure good health.

The animals were loaded into the experimental cart and the two catheters of each animal were flushed. The aortic line was used to continuously monitor pressure. During each experiment the animal received 10,000 U heparin/hour to prevent clot formation. Once a stable blood pressure was reached i.e., mean arterial pressure was maintained ±5mmHg for five minutes, the test began. The drug was then administered through the left atrial catheter with continual pressure monitoring. The time interval between drug tests was dependent on the animal and the dose previously given. The two preparations of pure recombinant kallikrein (rHK) designated as K-011 and K-012 were used in the tests. For K-011, assuming 1 mg rHK = 1.5 $A_{280}$, 0.25 ml solution used in the test is equivalent to the dosage of 2.5 µg rHK/Kg body weight, and for preparation K-012, 0.25 ml solution is equivalent to 1.25 µg rHK/Kg body weight.

The results are shown below in Tables XI - XIV. Even at a concentration as low as 1.25 µg/kg body weight can cause significant reduction in mean atrial pressure in the conscious pig (see Table XIV).

TABLE XI

| DRUG K-011 PIG 500 | | | | | | | |
|---|---|---|---|---|---|---|---|
| dose = 0.25 ml | | | | dose = 0.50 ml | | | |
| Time | HR | BP | MAP | Time | HR | BP | MAP |
| cont. | 115 | 170/115 | 137 | cont. | 105 | 165/115 | 132 |
| 30 sec | 210 | 175/100 | 125 | 30 sec | 165 | 140/70 | 93 |
| 1min | 175 | 175/100 | 125 | 1min | 147 | 155/70 | 98 |
| 2 min | 160 | 175/115 | 137 | 2 min | 125 | 160/110 | 127 |
| 3 min | 145 | 175/115 | 137 | 3 min | 120 | 160/110 | 127 |
| 4 min | 130 | 175/115 | 137 | 4 min | 120 | 160/110 | 127 |
| 5 min | 115 | 175/115 | 137 | 5 min | 120 | 160/110 | 127 |
| 6 min | 105 | 175/115 | 137 | 6 min | 120 | 160/110 | 127 |
| dose = 1.0 ml | | | | dose = 2.0ml | | | |
| Time | HR | BP | MAP | Time | HR | BP | MAP |
| cont. | 120 | 190/145 | 163 | cont. | 165 | 165/115 | 132 |
| 30 sec | 165 | 115/75 | 88 | 30 sec | 180 | 115/75 | 88 |
| 1 min | 165 | 155/70 | 127 | 1 min | 165 | 160/105 | 123 |
| 2 min | 160 | 160/110 | 117 | 2 min | 165 | 160/105 | 123 |
| 3 min | 165 | 165/115 | 132 | 3 min | 165 | 160/115 | 130 |
| 4 min | 165 | 165/115 | 132 | 4 min | 165 | 160/115 | 130 |
| 5 min | 165 | 165/115 | 132 | 5 min | 165 | 160/115 | 130 |
| 6 min | 165 | 165/115 | 132 | 6 min | 165 | 160/115 | 130 |
| cont. = control<br>HR = heart rate<br>BP = blood pressure<br>MAP = mean atrial pressure | | | | | | | |

TABLE XII

| DRUG K-011 PIG 14 | | | | | | | |
|---|---|---|---|---|---|---|---|
| dose = 0.25 ml | | | | dose = 0.50 ml | | | |
| Time | HR | BP | MAP | Time | HR | BP | MAP |
| cont. | 96 | 115/65 | 82 | cont. | 81 | 100/60 | 73 |
| 30 sec | 167 | 100/30 | 53 | 30 sec | 120 | 100/30 | 53 |
| 1 min | 120 | 100/30 | 53 | 1 min | 138 | 115/40 | 65 |
| 2 min | 96 | 100/35 | 55 | 2 min | 108 | 90/30 | 50 |
| 3 min | 90 | 100/45 | 63 | 3 min | 84 | 85/40 | 55 |
| 4 min | 90 | 110/60 | 77 | 4 min | 78 | 95/50 | 65 |
| 5 min | 78 | 100/65 | 77 | 5 min | 72 | 95/60 | 72 |
| 6 min | 80 | 105/75 | 85 | 6 min | 72 | 95/60 | 72 |
| dose = 1.0 ml | | | | dose = 2.0ml | | | |
| Time | HR | BP | MAP | Time | HR | BP | MAP |
| cont. | 90 | 100/60 | 163 | cont. | 72 | 95/60 | 72 |
| 30 sec | 138 | 95/35 | 88 | 30 sec | 138 | 80/25 | 43 |
| 1 min | 120 | 100/40 | 127 | 1 min | 120 | 100/45 | 63 |
| 2 min | 96 | 100/50 | 117 | 2 min | 96 | 95/55 | 68 |
| 3 min | 90 | 100/60 | 132 | 3 min | 96 | 100/60 | 73 |
| 4 min | 90 | 90/55 | 132 | 4 min | 84 | 100/70 | 80 |
| 5 min | 78 | 95/60 | 132 | 5 min | 90 | 100/70 | 80 |
| 6 min | 78 | 95/60 | 132 | 6 min | 72 | 105/70 | 82 |
| cont. = control<br>HR = heart rate<br>BP = blood pressure<br>MAP = mean atrial pressure | | | | | | | |

TABLE XIII

| DRUG K-012 PIG 14 | | | | | | | |
|---|---|---|---|---|---|---|---|
| dose = 0.25 ml | | | | dose = 0.50 ml | | | |
| Time | HR | BP | MAP | Time | HR | BP | MAP |
| cont. | 96 | 115/75 | 88 | cont. | 114 | 135/95 | 108 |
| 30 sec | 132 | 100/40 | 60 | 30 sec | 204 | 110/55 | 73 |
| 1 min | 115 | 105/65 | 78 | 1 min | 132 | 110/60 | 77 |
| 2 min | 104 | 110/75 | 86 | 2 min | 114 | 105/70 | 81 |
| 3 min | 104 | 110/75 | 86 | 3 min | 102 | 110/75 | 86 |
| 4 min | 104 | 110/75 | 86 | 4 min | 102 | 110/75 | 86 |
| 5 min | 104 | 110/75 | 86 | 5 min | 102 | 110/75 | 86 |
| 6 min | 104 | 110/75 | 86 | 6 min | 102 | 110/75 | 86 |
| dose = 1.0 ml | | | | | | | |
| Time | HR | BP | MAP | | | | |
| cont. | 96 | 110/75 | 86 | | | | |
| 30 sec | 168 | 100/40 | 60 | | | | |
| 1 min | 136 | 100/42.5 | 61.5 | | | | |
| 2 min | 102 | 100/50 | 67 | | | | |
| 3 min | 96 | 100/55 | 70 | | | | |
| 4 min | 90 | 100/65 | 77 | | | | |
| 5 min | 84 | 100/70 | 80 | | | | |
| 6 min | 84 | 100/70 | 80 | | | | |
| cont. = control<br>HR = heart rate<br>BP = blood pressure<br>MAP = mean atrial pressure | | | | | | | |

TABLE XIV

| DRUG K-012 PIG 43 | | | | | | | |
|---|---|---|---|---|---|---|---|
| dose = 2.0 | | | | dose = 4.0 | | | |
| Time | HR | BP | MAP | Time | HR | BP | MAP |
| cont. | 132 | 145/75 | 98 | cont. | 96 | 125/85 | 98 |
| 30 sec | 250 | 100/35 | 57 | 30 sec | 114 | 115/45 | 68 |
| 1min | 216 | 95/45 | 62 | 1min | 102 | 100/35 | 57 |
| 2 min | 144 | 90/50 | 63 | 2 min | 96 | 110/55 | 73 |
| 3 min | 120 | 90/60 | 70 | 3 min | 84 | 110/75 | 86 |
| 4 min | 120 | 120/85 | 97 | 4 min | 90 | 110/75 | 86 |
| 5 min | 96 | 125/85 | 98 | 5 min | 84 | 115/85 | 95 |
| 6 min | 96 | 135/100 | 112 | 6 min | 78 | 130/100 | 110 |
| cont. = control<br>HR = heart rate<br>BP = blood pressure<br>MAP = mean atrial pressure | | | | | | | |

The longest response obtained was six minutes.

Example 16

Kininogenase Activity and Esterase Activity of Purified Recombinant Human Kallikrein

Recombinant human kallikrein acted on the substrate kininogen to release kinin, i.e., kininogenase activity, an activity associated with naturally-occurring kallikrein. As represented in Table XV, the activity of the purified recombinant human mature kallikrein of the present invention was similar to the activity obtained from purified human urinary kallikrein.

Table XV

| | Kininogenase Specific Activity (1) | Esterase Unit (2) |
|---|---|---|
| Recombinant kallikrein | 29.0 | 45.1 |
| Human urinary kallikrein | 28.9 | 57.5 |
| Buffer alone | 0 | 0 |

(1) Kininogenase specific activity is expressed as µg kinin generated/30 min/mg kallikrein and is determined in accordance with the procedures of Example 12.
(2) Esterase unit is measured using: 3H- Tos-Arg-OMe as substrate and expressed as E.U./mg kallikrein and is determined in accordance with the procedures of Beaven et al., [Clin. Chim. Acta. 32, 67 (1971)].

The recombinant human kallikrein exhibited a dose response curve parallel to that of the purified human urinary kallikrein as measured by radioimmunoassay procedure of Example 7. This result indicates that the kallikrein produced by the recombinant cells have the same immunological property as naturally-occurring kallikrein produced by native cells.

The purified recombinant human mature kallikrein was analyzed in a 8-25% gradient sodium dodecyl sulfate-polyacrylamide gel and both recombinant human mature kallikrein, and naturally-occurring urinary kallikrein, have similar molecular weight a nd both showed heterogeneity in size (Figure 2). Size heterogeneity is common for glycoproteins due to the variation in the sugar chain length.

Example 17

Effect of Recombinant Human Kallikrein on Human Sperm Motility

Sperm were obtained from fertile normal donors. Purified human kallikrein (0.68 $A_{280}$) was added at the final dilution of 1:10 and 1:20 to 1-2x$10^6$ sperms in 100 µl final volume.
Sperm motility was measured using the procedure described by Mathur et al., Fertility and Sterility, Vol. 46, No. 3, 484(1986) and Mathur et al., American Journal of Reproductive Immunology and Microbiology, 12:87-89(1986) hereby incorporated by reference. The results from a 48hr observation were as follows:

| | % Motile |
|---|---|
| Control Media | 29.13 |
| r-HuK (1:10 dilution) | 42.76 |
| r-HuK (1:20 dilution) | 41.26 |

The recombinant kallikrein significantly enhanced the sperm motility. This biological activity of recombinant kallikrein is important as a therapeutic agent for treating male infertility.

Numerous modifications and variations in the practice of the invention are expected to occur to those skilled in the art upon consideration of the foregoing illustrative examples. Consequently, the invention should be considered as limited only to the extent reflected by the appended claims.

## Claims

### Claims for the following Contracting States : AT, BE, CH, LI, DE, FR, GB, IT, LU, NL, SE

1. A purified and isolated recombinantly derived kallikrein polypeptide comprising the amino acid sequence, extending from the N-terminus:

```
                                                      -1
                                 (Ala Pro Pro Ile Gln Ser Arg)n

+1                                  10                           20
Ile Val Gly Gly Trp Glu Cys Glu Gln His Ser Gln Pro Trp Gln Ala Ala Leu Tyr His

                                    30                           40
Phe Ser Thr Phe Gln Cys Gly Gly Ile Leu Val His Arg Gln Trp Val Leu Thr Ala Ala

                                    50                           60
His Cys Ile Ser Asp Asn Tyr Gln Leu Trp Leu Gly Arg His Asn Leu Phe Asp Asp Glu

                                    70                           80
Asn Thr Ala Gln Phe Val His Val Ser Glu Ser Phe Pro His Pro Gly Phe Asn Met Ser

                                    90                          100
Leu Leu Glu Asn His Thr Arg Gln Ala Asp Glu Asp Tyr Ser His Asp Leu Met Leu Leu

                                   110                          120
Arg Leu Thr Glu Pro Ala Asp Thr Ile Thr Asp Ala Val Lys Val Val Glu Leu Pro Thr

                                   130                          140
Gln Glu Pro Glu Val Gly Ser Thr Cys Leu Ala Ser Gly Trp Gly Ser Ile Glu Pro Glu

                                   150                          160
Asn Phe Ser Phe Pro Asp Asp Leu Gln Cys Val Asp Leu Lys Ile Leu Pro Asn Asp Glu

                                   170                          180
Cys Lys Lys Ala His Val Gln Lys Val Thr Asp Phe Met Leu Cys Val Gly His Leu Glu

                                   190                          200
Gly Gly Lys Asp Thr Cys Val Gly Asp Ser Gly Gly Pro Leu Met Cys Asp Gly Val Leu

                                   210                          220
Gln Gly Val Thr Ser Trp Gly Tyr Val Pro Cys Gly Thr Pro Asn Lys Pro Ser Val Ala

                                   230                      238
Val Arg Val Leu Ser Tyr Val Lys Trp Ile Glu Asp Thr Ile Ala Glu Asn Ser
```

wherein n is 0 or 1; and characterized by being the product of procaryotic or eucaryotic expression of an exogenous DNA sequence.

2. A purified and isolated recombinantly derived kallikrein polypeptide according to Claim 1 wherein n is 0.

3. A purified and isolated recombinantly derived kallikrein polypeptide according to Claim 1 wherein n is 1.

**4.** A purified and isolated recombinantly derived kallikrein polypeptide according to Claim 2 and free of association with any mammalian protein.

**5.** A purified and isolated recombinantly derived kallikrein polypeptide according to Claim 2 wherein the exogenous DNA sequence is a cDNA sequence.

**6.** A purified and isolated recombinantly derived kallikrein polypeptide according to Claim 2 wherein the exogenous DNA sequence is a manufactured DNA sequence.

**7.** A purified and isolated recombinantly derived kallikrein polypeptide according to Claim 2 wherein the exogenous DNA sequence is a genomic DNA sequence.

**8.** A purified and isolated recombinantly derived kallikrein polypeptide according to Claim 1 having a detectable label.

**9.** A purified and isolated DNA encoding for procaryotic or eucaryotic host expression of a kallikrein polypeptide of Claim 1.

**10.** A purified and isolated DNA according to Claim 9 having the nucleotide sequence set forth in Table V.

**11.** A purified and isolating DNA according to Claim 9 wherein the DNA is cDNA.

**12.** A purified and isolated DNA according to Claim 9 wherein the DNA is genomic DNA.

**13.** A purified and isolated DNA according to Claim 9 wherein the DNA is manufactured DNA.

**14.** A purified and isolated DNA according to Claim 13 having one or more codons preferred for expression in E. coli cells.

**15.** A purified and isolated DNA according to Claim 14 having the nucleotide sequence set forth in Table VI.

**16.** A pharmaceutical composition comprising a therapeutically effective amount of a kallikrein polypeptide of Claim 1 and pharmaceutically acceptable adjuvants.

**17.** Use of a kallikrein polypeptide according to Claim 1 for the manufacture of a medicament for providing vasodilation therapy.

**18.** Use of a kallikrein polypeptide according to Claim 1 for the manufacture of a medicament for treating male infertility.

**19.** A procaryotic or eucaryotic host cell transformed or transfected with DNA according to Claim 9 in a manner allowing the host cell to express a kallikrein polypeptide product of Claim 1.

**20.** A plasmid consisting of pDSHK1, wherein said plasmid is the ligation product of:
a) plasmid pDSVL digested with BamHI and end-filled; and
b) the DNA sequence shown in Table V extending from position 738 to position 5587.

**21.** A plasmid consisting of pDGHK-L1A, wherein said plasmid is the ligation product of:
a) a HindIII-EcoRI fragment consisting of nucleotides 2448 to 4362 of pBR322;
b) an EcoRI-PstI fragment from pMgI encoding an dihydrofolate reductase minigene wherein a deletion in the 3' intranslated region of the minigene is made by removing a 556 bp. Bg1II fragment and end-filling with Klenow enzyme;
c) a bidirectional SV40 termination sequence consisting of a BamHI-Bc1I fragment of necleotides 2553 to 2770 from SV40 DNA, having synthetic linker-1 added to the Bc1I end of the fragment to create a PstI site, and having synthetic linker-2 added to the BamHI end of the fragment to create a Sa1I site;

d) a kallikrein coding sequence consisting of nucleotides 801 to 5370 as shown in Table V having synthetic linker-3 added to the DraII site at position 801 and having a Sa1I site created by insertion of GA between nucleotides 15 and 16 3' to the TGA termination codon;
e) a rat glucose-regulated protein (GRP78) gene promoter consisting of SmaI-BsshII fragments of nucleotides -722 to -37 of the GRP78 sequence;
f) synthetic linker-4 wherein the BamHI site is ligated to the Bg1II site of linker-3; and
g) synthetic linker-5 wherein the SmaI site is added to the SmaI end of the GAP promoter sequence of (e) and the HindIII end added to the HindIII end of (a); and wherein the sequences of the linkers is as follows:

Linker 1:

```
    GCAATGGCAACAACGTTGCCCG
ACGTCGTTACCGTTGTTGCAACGGGCTAG
PstI                     (BclI)
```

Linker 2:

```
SalI   BamHI
TCGACTG
    GACCTAG
```

Linker 3:

```
BglII        (DraIII)
GATCTCAAACAGACAACAT
    AGTTTGTCTGTT
```

Linker 4:

```
CGCGCTCGATACTGGCTGTGACTACACTGACTTGGACACTTGGCCTTTTGCGGG
    GAGCTATGACCGACACTGATGTGACTGAACCTGTGAACCGGAAAACGCCC
BsshII
TTTGAG
AAACTCCTAG
      BamHI
```

Linker 5:

```
Hind III    EcoRI           SmaI
AGCTTGAGTCCTGAATTCGAGCTCGGTACCC
    ACTCAGGACTTAAGCTCGAGCCATGGG
```

**22.** A eucaryotic host cell transformed or transfected with a plasmid of Claim 20 or 21.

**23.** A method of producing a purified and isolated kallikrein polypeptide comprising the steps of:
transfecting or transforming host cells with DNA according to Claim 9;
culturing the transfected or transformed host cells to allow the host cells to express kallikrein polypeptide; and isolating kallikrein.

**Claims for the following Contracting States : ES, GR**

**1.** A method of producing a purified and isolated recombinantly derived kallikrein polypeptide comprising the amino acid sequence, extending from the N-terminus:

(-7 ... -1): (Ala Pro Pro Ile Gln Ser Arg)n

+1: Ile Val Gly Gly Trp Glu Cys Glu Gln His (10) Ser Gln Pro Trp Gln Ala Ala Leu Tyr His (20)

Phe Ser Thr Phe Gln Cys Gly Gly Ile Leu (30) Val His Arg Gln Trp Val Leu Thr Ala Ala (40)

His Cys Ile Ser Asp Asn Tyr Gln Leu Trp (50) Leu Gly Arg His Asn Leu Phe Asp Asp Glu (60)

Asn Thr Ala Gln Phe Val His Val Ser Glu (70) Ser Phe Pro His Pro Gly Phe Asn Met Ser (80)

Leu Leu Glu Asn His Thr Arg Gln Ala Asp (90) Glu Asp Tyr Ser His Asp Leu Met Leu Leu (100)

Arg Leu Thr Glu Pro Ala Asp Thr Ile Thr (110) Asp Ala Val Lys Val Val Glu Leu Pro Thr (120)

Gln Glu Pro Glu Val Gly Ser Thr Cys Leu (130) Ala Ser Gly Trp Gly Ser Ile Glu Pro Glu (140)

Asn Phe Ser Phe Pro Asp Asp Leu Gln Cys (150) Val Asp Leu Lys Ile Leu Pro Asn Asp Glu (160)

Cys Lys Lys Ala His Val Gln Lys Val Thr (170) Asp Phe Met Leu Cys Val Gly His Leu Glu (180)

Gly Gly Lys Asp Thr Cys Val Gly Asp Ser (190) Gly Gly Pro Leu Met Cys Asp Gly Val Leu (200)

Gln Gly Val Thr Ser Trp Gly Tyr Val Pro (210) Cys Gly Thr Pro Asn Lys Pro Ser Val Ala (220)

Val Arg Val Leu Ser Tyr Val Lys Trp Ile (230) Glu Asp Thr Ile Ala Glu Asn Ser (238)

wherein n is 0 or 1; and characterized by being the product of procaryotic or eucaryotic expression of an exogenous DNA sequence, which method comprises expressing said DNA sequence in procaryotic or eucaryotic calls to produce said polypeptide.

**2.** A method of producing a purified and isolated recombinantly derived kallikrein polypeptide according to Claim 1 wherein n is 0.

**3.** A method of producing a purified and isolated recombinantly derived kallikrein polypeptide according to claim 1 wherein n is 1.

**4.** A method of producing a purified and isolated recombinantly derived kallikrein polypeptide according to claim 2 and free of association with any mammalian protein.

**5.** A method of producing a purified and isolated recombinantly derived kallikrein polypeptide according to Claim 2 wherein the exogenous DNA sequence is a cDNA sequence.

**6.** A method of producing a purified and isolated recombinantly derived kallikrein polypeptide according to claim 2 wherein the exogenous DNA sequence is a manufactured DNA sequence.

**7.** A method of producing a purified and isolated recombinantly derived kallikrein polypeptide according to Claim 2 wherein the exogenous DNA sequence is a genomic DNA sequence.

**8.** A method of producing a purified and isolated recombinantly derived kallikrein polypeptide according to Claim 1 having a detectable label.

**9.** A method of producing a purified and isolated DNA encoding for procaryotic or eucaryotic host expression of a kallikrein polypeptide producible according to Claim 1 which method comprises purifying and isolating said DNA.

**10.** A method of producing a purified and isolated DNA according to Claim 9 having the nucleotide sequence set forth in Table V.

**11.** A method of producing a purified and isolating DNA according to Claim 9 wherein the DNA is cDNA.

**12.** A method of producing a purified and isolated DNA according to claim 9 wherein the DNA is genomic DNA.

**13.** A method of producing a purified and isolated DNA according to Claim 9 wherein the DNA is manufactured DNA.

**14.** A method of producing a purified and isolated DNA according to Claim 13 having one or more codons preferred for expression in E. coli cells.

**15.** A method of producing a purified and isolated DNA according to claim 14 having the nucleotide sequence set forth in Table VI.

**16.** A method of producing a pharmaceutical composition, which method comprises combining a therapeutically effective amount of a kallikrein polypeptide producible according to Claim 1 and a pharmaceutically acceptable adjuvant.

**17.** Use of a kallikrein polypeptide producible according to Claim 1 for the manufacture of a medicament for providing vasodilation therapy.

**18.** Use of a kallikrein polypeptide producible according to Claim 1 for the manufacture of a medicament for treating male infertility.

**19.** A method of producing a procaryotic or eucaryotic host cell which method comprises transforming or transfecting a procaryotic or eucaryotic cell with DNA producible according to Claim 9 in a manner allowing the host cell to express a kallikrein polypeptide product producible according to Claim 1.

**20.** A method of producing a plasmid consisting of pDSHK1, comprising ligating:
a) plasmid pDSVL digested with BamHI and end-filled; and
b) the DNA sequence shown in Table V extending from position 738 to position 5587.

**21.** A method of producing a plasmid consisting of pDGHK-L1A, comprising ligating:
a) a HindIII-EcoRI fragment consisting of nucleotides 2448 to 4362 of pBR322;
b) an EcoRI-PstI fragment from pMgI encoding an dihydrofolate reductase minigene wherein a deletion in the 3' intranslated region of the minigene is made by removing a 556 bp. Bg1II fragment and end-filling with Klenow enzyme;

c) a bidirectional SV40 termination sequence consisting of a BamHI-Bc1I fragment of necleotides 2553 to 2770 from SV40 DNA, having synthetic linker-1 added to the Bc1I end of the fragment to create a PstI site, and having synthetic linker-2 added to the BamHI end of the fragment to create a Sa1I site;

d) a kallikrein coding sequence consisting of nucleotides 801 to 5370 as shown in Table V having synthetic linker-3 added to the DraIII site at position 801 and having a Sa1I site created by insertion of GA between nucleotides 15 and 16 3' to the TGA termination codon;

e) a rat glucose-regulated protein (GRP78) gene promoter consisting of SmaI-BsshII fragments of nucleotides -722 to -37 of the GRP78 sequence;

f) synthetic linker-4 wherein the BamHI site is ligated to the Bg1II site of linker-3; and

g) synthetic linker-5 wherein the SmaI site is added to the SmaI end of the GRP promoter sequence of (e) and the HindIII end added to the HindIII end of (a); and wherein the sequences of the linkers is as follows:

Linker 1:

```
         GCAATGGCAACAACGTTGCCCG
ACGTCGTTACCGTTGTTGCAACGGGCCTAG
PstI                        (BclI)
```

Linker 2:

```
SalI  BamHI
TCGACTG
    GACCTAG
```

Linker 3:

```
BglII        (DraIII)
GATCTCAAACAGACAACAT
    AGTTTCTCTGTT
```

Linker 4:

```
CGCGCTCGATACTCCCTGTGACTACACTGACTTGGACACTTGGCCTTTTGCGGG
    GAGCTATGACCGACACTGATGTCACTGAACCTGTGAACCGGAAAACGCCC
BsshII
TTTGAG
AAACTCCTAG
     BamHI
```

Linker 5:

```
Hind III    EcoRI          SmaI
AGCTTGAGTCCTGAATTCGAGCTCGGTACCC
    ACTCAGGACTTAAGCTCGAGCCATGGG
```

22. A method of producing a eucaryotic host cell comprising transforming or transfecting a eucaryotic cell with a plasmid producible according to Claim 20 or 21.

23. A method of producing a purified and isolated kallikrein polypeptide comprising the steps of:
transfecting or transforming host cells with DNA according to Claim 9;
culturing the transfected or transformed host cells to allow the host cells to express kallikrein polypeptide; and isolating kallikrein.

**Patentansprüche**

**Patentansprüche für folgende Vertragsstaaten : AT, BE, CH, LI, DE, FR, GB, IT, LU, NL, SE**

1. Ein gereinigtes und isoliertes, rekombinant gewonnenes Kallikrein-Polypeptid, das die sich vom N-Terminus aus erstreckende Aminosäuresequenz:

(Ala Pro Pro Ile Gln Ser Arg$^{-1}$)$_n$

+1 Ile Val Gly Gly Trp Glu Cys Glu Gln His (10) Ser Gln Pro Trp Gln Ala Ala Leu Tyr His (20)

Phe Ser Thr Phe Gln Cys Gly Gly Ile Leu (30) Val His Arg Gln Trp Val Leu Thr Ala Ala (40)

His Cys Ile Ser Asp Asn Tyr Gln Leu Trp (50) Leu Gly Arg His Asn Leu Phe Asp Asp Glu (60)

Asn Thr Ala Gln Phe Val His Val Ser Glu (70) Ser Phe Pro His Pro Gly Phe Asn Met Ser (80)

Leu Leu Glu Asn His Thr Arg Gln Ala Asp (90) Glu Asp Tyr Ser His Asp Leu Met Leu Leu (100)

Arg Leu Thr Glu Pro Ala Asp Thr Ile Thr (110) Asp Ala Val Lys Val Val Glu Leu Pro Thr (120)

Gln Glu Pro Glu Val Gly Ser Thr Cys Leu (130) Ala Ser Gly Trp Gly Ser Ile Glu Pro Glu (140)

Asn Phe Ser Phe Pro Asp Asp Leu Gln Cys (150) Val Asp Leu Lys Ile Leu Pro Asn Asp Glu (160)

Cys Lys Lys Ala His Val Gln Lys Val Thr (170) Asp Phe Met Leu Cys Val Gly His Leu Glu (180)

Gly Gly Lys Asp Thr Cys Val Gly Asp Ser (190) Gly Gly Pro Leu Met Cys Asp Gly Val Leu (200)

Gln Gly Val Thr Ser Trp Gly Tyr Val Pro (210) Cys Gly Thr Pro Asn Lys Pro Ser Val Ala (220)

Val Arg Val Leu Ser Tyr Val Lys Trp Ile (230) Glu Asp Thr Ile Ala Glu Asn Ser (238)

umfaßt, worin n 0 oder 1 ist; und dadurch gekennzeichnet, daß es das Produkt prokaryontischer oder eukaryontischer Expression einer exogenen DNA-Sequenz ist.

2. Ein gereinigtes und isoliertes, rekombinant gewonnenes Kallikrein-Polypeptid nach Anspruch 1, worin n 0 ist.

3. Ein gereinigtes und isoliertes, rekombinant gewonnenes Kallikrein-Polypeptid nach Anspruch 1, worin n 1 ist.

4. Ein gereinigtes und isoliertes, rekombinant gewonnenes Kallikrein-Polypeptid nach Anspruch 2 und frei von Assoziation mit irgendeinem Säugetier-Protein.

5. Ein gereinigtes und isoliertes, rekombinant gewonnenes Kallikrein-Polypeptid nach Anspruch 2, wobei die exogene DNA-Sequenz eine cDNA-Sequenz ist.

6. Ein gereinigtes und isoliertes, rekombinant gewonnenes Kallikrein-Polypeptid nach Anspruch 2, wobei die exogene DNA-Sequenz eine künstlich hergestellte DNA-Sequenz ist.

7. Ein gereinigtes und isoliertes, rekombinant gewonnenes Kallikrein-Polypeptid nach Anspruch 2, wobei die exogene DNA-Sequenz eine Genom-DNA-Sequenz ist.

8. Ein gereinigtes und isoliertes, rekombinant gewonnenes Kallikrein-Polypeptid nach Anspruch 1, mit einer nachweisbaren Markierung.

9. Eine gereinigte und isolierte DNA, die für die Expression eines Kallikrein-Polypeptids nach Anspruch 1 in einem prokaryontischen oder eukaryontischen Wirt kodiert.

10. Eine gereinigte und isolierte DNA nach Anspruch 9, mit der Nukleotidsequenz, die in Tabelle V angegeben ist.

11. Eine gereinigte und isolierte DNA nach Anspruch 9, wobei die DNA cDNA ist.

12. Eine gereinigte und isolierte DNA nach Anspruch 9, wobei die DNA Genom-DNA ist.

13. Eine gereinigte und isolierte DNA nach Anspruch 9, wobei die DNA künstlich hergestellte DNA ist.

14. Eine gereinigte und isolierte DNA nach Anspruch 13, mit einem oder mehreren Codons, die für Expression in E. coli-Zellen bevorzugt sind.

15. Eine gereinigte und isolierte DNA nach Anspruch 14, mit der Nukleotidsequenz, die in Tabelle VI angegeben ist.

16. Eine pharmazeutische Zusammensetzung, die eine therapeutisch wirksame Menge eines Kallikrein-Polypeptids nach Anspruch 1 und pharmazeutisch annehmbare Hilfsstoffe umfaßt.

17. Verwendung eines Kallikrein-Polypeptids nach Anspruch 1 zur Herstellung eines Arzneimittels zur Bereitstellung von Vasodilatationstherapie.

18. Verwendung eines Kallikrein-Polypeptids nach Anspruch 1 zur Herstellung eines Arzneimittels zur Behandlung von Unfruchtbarkeit beim Manne.

19. Eine prokaryontische oder eukaryontische Wirtszelle, die mit DNA nach Anspruch 9 in einer Weise transformiert oder transfiziert ist, die es der Wirtszelle ermöglicht, ein Kallikrein-Polypeptidprodukt nach Anspruch 1 zu exprimieren.

20. Ein Plasmid, das aus pDSHK1 besteht, wobei besagtes Plasmid das Ligationsprodukt aus:
   a) Plasmid pDSVL, verdaut mit BamHI und end-gefüllt; und
   b) der in Tabelle V dargestellten DNA-Sequenz, die sich von Position 738 bis Position 5587 erstreckt,

ist.

**21.** Ein Plasmid, das aus pDGHK-L1A besteht, wobei besagtes Plasmid das Ligationsprodukt aus:

a) einem HindIII-EcoRI-Fragment, das aus den Nukleotiden 2448 bis 4362 von pBR322 besteht;

b) einem EcoRI-PstI-Fragment aus pMgI, das ein Dihydrofolat-Reduktase-Minigen kodiert, wobei eine Deletion in der 3' nicht-translierten Region des Minigens vorgenommen ist, indem ein 556 bp langes BglII-Fragment entfernt und mit Klenow-Enzym end-gefüllt worden ist;

c) einer bidirektionalen SV40-Terminationssequenz, bestehend aus einem BamHI-BclI-Fragment der Nukleotide 2553 bis 2770 aus SV40-DNA, mit synthetischem Linker-1, der zum BclI-Ende des Fragments hinzugefügt ist, um eine PstI-Stelle zu schaffen, und mit synthetischem Linker-2, der zum BamHI-Ende des Fragments hinzugefügt ist, um eine SalI-Stelle zu schaffen;

d) einer Kallikrein-Kodierungssequenz, bestehend aus den Nukleotiden 801 bis 5370, wie dargestellt in Tabelle V, mit synthetischem Linker-3, der zur DraIII-Stelle an Position 801 hinzugefügt ist, und mit einer SalI-Stelle, die durch Insertion von GA zwischen den Nukleotiden 15 und 16 3' zum TGA-Terminationscodon geschaffen ist;

e) einem Glucose-regulierten Ratten-Protein(GRP78)-Genpromotor, bestehend aus SmaI-BsshII-Fragmenten der Nukleotide -722 bis -37 der GRP78-Sequenz;

f) synthetischem Linker-4, wobei die BamHI-Stelle an die BglII-Stelle von Linker-3 ligiert ist; und

g) synthetischem Linker-5, wobei die SmaI-Stelle zum SmaI-Ende der GRP-Promotorsequenz von (e) hinzugefügt ist und das HindII-Ende zum HindIII-Ende von (a) hinzugefügt ist;

ist und wobei die Sequenzen der Linker wie folgt sind:

```
Linker 1:
            GCAATGGCAACAACGTTGCCCG
ACGTCGTTACCGTTGTTGCAACGGGCCTAG
PstI                       (BclI)

Linker 2:
SalI   BamHI
TCGACTG
   GACCTAG

Linker 3:
BglII         (DraIII)
GATCTCAAACAGACAACAT
   AGTTTGTCTGTT

Linker 4:
CGCGCTCGATACTGGCTGTGACTACACTGACTTGGACACTTGGCCTTTTGCGGG
   GAGCTATGACCGACACTGATGTGACTGAACCTGTGAACCGGAAAACGCCC
BsshII
TTTGAG
AAACTCCTAG
     BamHI
Linker 5:
Hind III    EcoRI          SmaI
AGCTTGAGTCCTGAATTCGAGCTCGGTACCC
   ACTCAGGACTTAAGCTCGAGCCATGGG
```

22. Eine eukaryontische Wirtszelle, transformiert oder transfiziert mit einem Plasmid nach Anspruch 20 oder 21.

23. Ein Verfahren zur Herstellung eines gereinigten und isolierten Kallikrein-Polypeptids, das die Schritte umfaßt:
Transfizieren oder Transformieren von Wirtszellen mit DNA nach Anspruch 9;
Kultivieren der transfizierten oder transformierten Wirtszellen, um den Wirtszellen zu ermöglichen, Kallikrein-Polypeptid zu exprimieren;
und Isolieren von Kallikrein.

**Patentansprüche für folgende Vertragsstaaten : ES, GR**

1. Ein Verfahren zur Herstellung eines gereinigten und isolierten, rekombinant gewonnenen Kallikrein-Polypeptids, das die sich vom N-Terminus aus erstreckende Aminosäuresequenz:

-1
(Ala Pro Pro Ile Gln Ser Arg)n

+1 10 20
Ile Val Gly Gly Trp Glu Cys Glu Gln His Ser Gln Pro Trp Gln Ala Ala Leu Tyr His

30 40
Phe Ser Thr Phe Gln Cys Gly Gly Ile Leu Val His Arg Gln Trp Val Leu Thr Ala Ala

50 60
His Cys Ile Ser Asp Asn Tyr Gln Leu Trp Leu Gly Arg His Asn Leu Phe Asp Asp Glu

70 80
Asn Thr Ala Gln Phe Val His Val Ser Glu Ser Phe Pro His Pro Gly Phe Asn Met Ser

90 100
Leu Leu Glu Asn His Thr Arg Gln Ala Asp Glu Asp Tyr Ser His Asp Leu Met Leu Leu

110 120
Arg Leu Thr Glu Pro Ala Asp Thr Ile Thr Asp Ala Val Lys Val Val Glu Leu Pro Thr

130 140
Gln Glu Pro Glu Val Gly Ser Thr Cys Leu Ala Ser Gly Trp Gly Ser Ile Glu Pro Glu

150 160
Asn Phe Ser Phe Pro Asp Asp Leu Gln Cys Val Asp Leu Lys Ile Leu Pro Asn Asp Glu

170 180
Cys Lys Lys Ala His Val Gln Lys Val Thr Asp Phe Met Leu Cys Val Gly His Leu Glu

190 200
Gly Gly Lys Asp Thr Cys Val Gly Asp Ser Gly Gly Pro Leu Met Cys Asp Gly Val Leu

210 220
Gln Gly Val Thr Ser Trp Gly Tyr Val Pro Cys Gly Thr Pro Asn Lys Pro Ser Val Ala

230 238
Val Arg Val Leu Ser Tyr Val Lys Trp Ile Glu Asp Thr Ile Ala Glu Asp Ser

umfaßt, worin n 0 oder 1 ist; und dadurch gekennzeichnet, daß es das Produkt prokaryontischer oder eukaryontischer Expression einer exogenen DNA-Sequenz ist, wobei das Verfahren das Exprimieren besagter DNA-Sequenz in prokaryontischen und eukaryontischen Zellen umfaßt, um besagtes Polypeptid herzustellen.

**2.** Ein Verfahren zur Herstellung eines gereinigten und isolierten, rekombinant gewonnenen Kallikrein-Polypeptids nach Anspruch 1, worin n 0 ist.

**3.** Ein Verfahren zur Herstellung eines gereinigten und isolierten, rekombinant gewonnenen Kallikrein-Polypeptids nach Anspruch 1, worin n 1 ist.

**4.** Ein Verfahren zur Herstellung eines gereinigten und isolierten, rekombinant gewonnenen Kallikrein-Polypeptids nach Anspruch 2 und frei von Assoziation mit irgendeinem Säugetier-Protein.

**5.** Ein Verfahren zur Herstellung eines gereinigten und isolierten, rekombinant gewonnenen Kallikrein-Polypeptids nach Anspruch 2, wobei die exogene DNA-Sequenz eine cDNA-Sequenz ist.

**6.** Ein Verfahren zur Herstellung eines gereinigten und isolierten, rekombinant gewonnenen Kallikrein-Polypeptids nach Anspruch 2, wobei die exogene DNA-Sequenz eine künstlich hergestellte DNA-Sequenz ist.

**7.** Ein Verfahren zur Herstellung eines gereinigten und isolierten, rekombinant gewonnenen Kallikrein-Polypeptids nach Anspruch 2, wobei die exogene DNA-Sequenz eine Genom-DNA-Sequenz ist.

**8.** Ein Verfahren zur Herstellung eines gereinigten und isolierten, rekombinant gewonnenen Kallikrein-Polypeptids nach Anspruch 1, mit einer nachweisbaren Markierung.

**9.** Ein Verfahren zur Herstellung einer gereinigten und isolierten DNA, die für die Expression eines Kallikrein-Polypeptids, herstellbar nach Anspruch 1, in einem prokaryontischen oder eukaryontischen Wirt kodiert, wobei das Verfahren die Reinigung und Isolierung besagter DNA umfaßt.

**10.** Ein Verfahren zur Herstellung einer gereinigten und isolierten DNA nach Anspruch 9, mit der Nukleotidsequenz, die in Tabelle V angegeben ist.

**11.** Ein Verfahren zur Herstellung einer gereinigten und isolierten DNA nach Anspruch 9, wobei die DNA cDNA ist.

**12.** Ein Verfahren zur Herstellung einer gereinigten und isolierten DNA nach Anspruch 9, wobei die DNA Genom-DNA ist.

**13.** Ein Verfahren zur Herstellung einer gereinigten und isolierten DNA nach Anspruch 9, wobei die DNA künstlich hergestellte DNA ist.

**14.** Ein Verfahren zur Herstellung einer gereinigten und isolierten DNA nach Anspruch 13, mit einem oder mehreren Codons, die für Expression in E. coli-Zellen bevorzugt sind.

**15.** Ein Verfahren zur Herstellung einer gereinigten und isolierten DNA nach Anspruch 14, mit der Nukleotidsequenz, die in Tabelle VI angegeben ist.

**16.** Ein Verfahren zur Herstellung einer pharmazeutischen Zusammensetzung, wobei das Verfahren das Kombinieren einer therapeutisch wirksamen Menge eines Kallikrein-Polypeptids, herstellbar nach Anspruch 1, und eines pharmazeutisch annehmbaren Hilfsstoffes umfaßt.

**17.** Verwendung eines Kallikrein-Polypeptids, herstellbar nach Anspruch 1, zur Herstellung eines Arzneimittels zur Bereitstellung von Vasodilatationstherapie.

**18.** Verwendung eines Kallikrein-Polypeptids, herstellbar nach Anspruch 1, zur Herstellung eines Arzneimittels zur Behandlung von Unfruchtbarkeit beim Manne.

**19.** Ein Verfahren zur Herstellung einer prokaryontischen oder eukaryontischen Wirtszelle, wobei das Verfahren das Transformieren oder Transfizieren einer prokaryontischen oder eukaryontischen Zelle mit DNA, die nach Anspruch 9 herstellbar ist, in einer Weise umfaßt, die es der Wirtszelle ermöglicht, ein Kallikrein-Polypeptidprodukt, das nach Anspruch 1 herstellbar ist, zu exprimieren.

**20.** Ein Verfahren zur Herstellung eines Plasmids, das aus pDSHK1 besteht, welches das Ligieren von:
a) Plasmid pDSVL, verdaut mit BamHI und end-gefüllt; und
b) der in Tabelle V dargestellten DNA-Sequenz, die sich von Position 738 bis Position 5587 erstreckt,
umfaßt.

**21.** Ein Verfahren zur Herstellung eines Plasmids, das aus pDGHK-L1A besteht, welches das Ligieren von:
a) einem HindIII-EcoRI-Fragment, das aus den Nukleotiden 2448 bis 4362 von pBR322 besteht;

b) einem EcoRI-PstI-Fragment aus pMgI, das ein Dihydrofolat-Reduktase-Minigen kodiert, wobei eine Deletion in der 3' nicht-translierten Region des Minigens vorgenommen ist, indem ein 556 bp langes BglII-Fragment entfernt und mit Klenow-Enzym end-gefüllt worden ist;
c) einer bidirektionalen SV40-Terminationssequenz, bestehend aus einem BamHI-BclI-Fragment der Nukleotide 2553 bis 2770 aus SV40-DNA, mit synthetischem Linker-1, der zum BclI-Ende des Fragments hinzugefügt ist, um eine PstI-Stelle zu schaffen, und mit synthetischem Linker-2, der zum BamHI-Ende des Fragments hinzugefügt ist, um eine SalI-Stelle zu schaffen;
d) einer Kallikrein-Kodierungssequenz, bestehend aus den Nukleotiden 801 bis 5370, wie dargestellt in Tabelle V, mit synthetischem Linker-3, der zur DraIII-Stelle an Position 801 hinzugefügt ist, und mit einer SalI-Stelle, die durch Insertion von GA zwischen den Nukleotiden 15 und 16 3' zum TGA-Terminationscodon geschaffen ist;
e) einem Glucose-regulierten Ratten-Protein(GRP78)-Genpromotor, bestehend aus SmaI-BsshII-Fragmenten der Nukleotide -722 bis -37 der GRP78-Sequenz;
f) synthetischem Linker-4, wobei die BamHI-Stelle an die BglII-Stelle von Linker-3 ligiert ist; und
g) synthetischem Linker-5, wobei die SmaI-Stelle zum SmaI-Ende der GRP-Promotorsequenz von (e) hinzugefügt ist und das HindII-Ende zum HindIII-Ende von (a) hinzugefügt ist;
umfaßt und wobei die Sequenzen der Linker wie folgt sind:

<u>Linker 1</u>:

```
             GCAATGGCAACAACGTTGCCCG
ACGTCGTTACCGTTGTTGCAACGGGCCTAG
PstI                        (BclI)
```

<u>Linker 2</u>:

```
SalI  BamHI
TCGACTG
   GACCTAG
```

<u>Linker 3</u>:

```
BglII        (DraIII)
GATCTCAAACAGACAACAT
   AGTTTGTCTGTT
```

<u>Linker 4</u>:

```
CGCGCTCGATACTGGCTGTGACTACACTGACTTGGACACTTGGCCTTTTGCGGG
   GAGCTATGACCGACACTGATGTGACTGAACCTGTGAACCGGAAAACGCCC
BsshII
TTTGAG
AAACTCCTAG
     BamHI
```

<u>Linker 5</u>:

```
Hind III    EcoRI          SmaI
AGCTTGAGTCCTGAATTCGAGCTCGGTACCC
   ACTCAGGACTTAAGCTCGAGCCATGGG
```

**22.** Ein Verfahren zur Herstellung einer eukaryontischen Wirtszelle, welches das Transformieren oder Transfizieren einer eukaryontischen Zelle mit einem gemäß Anspruch 20 oder 21 herstellbaren Plasmid umfaßt.

**23.** Ein Verfahren zur Herstellung eines gereinigten und isolierten Kallikrein-Polypeptids, das die Schritte umfaßt:
Transfizieren oder Transformieren von Wirtszellen mit DNA nach Anspruch 9;
Kultivieren der transfizierten oder transformierten Wirtszellen, um den Wirtszellen zu ermöglichen, Kallikrein-Polypeptid zu exprimieren;
und Isolieren von Kallikrein.

**Revendications**
**Revendications pour les Etats contractants suivants : AT, BE, CH, LI, DE, FR, GB, IT, LU, NL, SE**

**1.** Polypeptide de kallikréine, dérivé de façon recombinante, purifié et isolé, comprenant la séquence d'acides aminés, s'étendant à partir de la terminaison N :

-1
(Ala Pro Pro Ile Gln Ser Arg)n

+1 10 20
Ile Val Gly Gly Trp Glu Cys Glu Gln His Ser Gln Pro Trp Gln Ala Ala Leu Tyr His

30 40
Phe Ser Thr Phe Gln Cys Gly Gly Ile Leu Val His Arg Gln Trp Val Leu Thr Ala Ala

50 60
His Cys Ile Ser Asp Asn Tyr Gln Leu Trp Leu Gly Arg His Asn Leu Phe Asp Asp Glu

70 80
Asn Thr Ala Gln Phe Val His Val Ser Glu Ser Phe Pro His Pro Gly Phe Asn Met Ser

90 100
Leu Leu Glu Asn His Thr Arg Gln Ala Asp Glu Asp Tyr Ser His Asp Leu Met Leu Leu

110 120
Arg Leu Thr Glu Pro Ala Asp Thr Ile Thr Asp Ala Val Lys Val Val Glu Leu Pro Thr

130 140
Gln Glu Pro Glu Val Gly Ser Thr Cys Leu Ala Ser Gly Trp Gly Ser Ile Glu Pro Glu

150 160
Asn Phe Ser Phe Pro Asp Asp Leu Gln Cys Val Asp Leu Lys Ile Leu Pro Asn Asp Glu

170 180
Cys Lys Lys Ala His Val Gln Lys Val Thr Asp Phe Met Leu Cys Val Gly His Leu Glu

190 200
Gly Gly Lys Asp Thr Cys Val Gly Asp Ser Gly Gly Pro Leu Met Cys Asp Gly Val Leu

210 220
Gln Gly Val Thr Ser Trp Gly Tyr Val Pro Cys Gly Thr Pro Asn Lys Pro Ser Val Ala

230 238
Val Arg Val Leu Ser Tyr Val Lys Trp Ile Glu Asp Thr Ile Ala Glu Asn Ser

dans laquelle n est 0 ou 1 ; et caractérisé par le fait d'être le produit de l'expression procaryote ou eucaryote d'une séquence d'ADN exogène.

**2.** Polypeptide de kallikréine, dérivé de façon recombinante, purifié et isolé selon la revendication 1, dans lequel n est 0.

**3.** Polypeptide de kallikréine, dérivé de façon recombinante, purifié et isolé selon la revendication 1, dans lequel n est 1.

**4.** Polypeptide de kallikréine, dérivé de façon recombinante, purifié et isolé selon la revendication 2 et dépourvu d'association avec une protéine quelconque de mammifère.

**5.** Polypeptide de kallikréine, dérivé de façon recombinante, purifié et isolé selon la revendication 2, dans lequel la séquence d'ADN exogène est une séquence d'ADNc.

**6.** Polypeptide de kallikréine, dérivé de façon recombinante, purifié et isolé selon la revendication 2, dans lequel la séquence d'ADN exogène est une séquence d'ADN fabriqué.

**7.** Polypeptide de kallikréine, dérivé de façon recombinante, purifié et isolé selon la revendication 2, dans lequel la séquence d'ADN exogène est une séquence d'ADN génomique.

**8.** Polypeptide de kallikréine, dérivé de façon recombinante, purifié et isolé selon la revendication 1, ayant un marqueur détectable.

**9.** ADN purifié et isolé codant pour l'expression par un hôte procaryote ou eucaryote d'un polypeptide de kallikréine de la revendication 1.

**10.** ADN isolé et purifié selon la revendication 9 ayant la séquence de nucléotides indiquée dans le tableau V.

**11.** ADN purifié et isolé selon la revendication 9, dans lequel l'ADN est de l'ADNc.

**12.** ADN purifié et isolé selon la revendication 9, dans lequel l'ADN est de l'ADN génomique.

**13.** ADN purifié et isolé selon la revendication 9, dans lequel l'ADN est de l'ADN fabriqué.

**14.** ADN purifié et isolé selon la revendication 13, ayant un ou plusieurs codons préférés pour l'expression dans des cellules de E. coli.

**15.** ADN purifié et isolé selon la revendication 14, ayant la séquence de nucléotides indiquée dans le tableau VI.

**16.** Composition pharmaceutique comprenant une quantité thérapeutiquement efficace d'un polypeptide de kallikréine de la revendication 1 et des adjuvants pharmaceutiquement acceptables.

**17.** Utilisation d'un polypeptide de kallikréine selon la revendication 1 pour la fabrication d'un médicament pour fournir une thérapie de vasodilatation.

**18.** Utilisation d'un polypeptide de kallikréine selon la revendication 1 pour la fabrication d'un médicament pour traiter la stérilité masculine.

**19.** Cellule hôte procaryote ou eucaryote transformée ou transfectée par de l'ADN selon la revendication 9 d'une façon permettant à la cellule hôte d'exprimer un polypeptide de kallikréine de la revendication 1.

**20.** Plasmide constitué de pDSHK1, dans lequel ledit plasmide est le produit de ligature de :
a) le plasmide pDSVL digéré par BamHI et rempli aux extrémités ; et
b) la séquence d'ADN montrée dans le tableau V s'étendant de la position 738 à la position 5587.

**21.** Plasmide constitué de pDGHK-L1A, dans lequel ledit plasmide est le produit de ligature de :

a) un fragment HindIII-EcoRI constitué des nucléotides 2448 à 4362 de pBR322 ;

b) un fragment EcoRI-PstI de pMgI codant un minigène de réductase de dihydrofolate, dans lequel une délétion dans la région non traduite 3' du minigène est réalisée en retirant un fragment BglII de 556 paires de bases et en remplissant aux extrémités par une enzyme de Klenow ;

c) une séquence de terminaison SV40 bidirectionnelle constituée d'un fragment BamHI-BclI des nucléotides 2553 à 2770 d'ADN SV40, ayant un lieur-1 synthétique ajouté à l'extrémité BclI du fragment pour créer un site PstI, et ayant un lieur-2 synthétique ajouté à l'extrémité BamHI du fragment pour créer un site SalI ;

d) une séquence codant la kallikréine constituée des nucléotides 801 à 5370 comme montré dans le tableau V ayant un lieur-3 synthétique ajouté au site DraIII en position 801 et ayant un site SalI créé par insertion de GA entre les nucléotides 15 et 16 3' au codon de terminaison TGA ;

e) un promoteur du gène de la protéine de régulation du glucose chez les rats (GRP78), constitué des fragments SmaI-BsshII des nucléotides -722 à -37 de la séquence GRP78 ;

f) un lieur-4 synthétique dans lequel le site BamHI est ligaturé au site BglII du lieur-3 ; et

g) un lieur-5 synthétique, dans lequel le site SmaI est ajouté à l'extrémité SmaI de la séquence de promoteur GRP de (e) et l'extrémité HindIII ajoutée à l'extrémité HindIII de (a) ; et dans lequel les séquences des lieurs sont comme suit :

Lieur 1

```
          GCAATGGCAACAACGTTGCCCG
ACGTCGTTACCGTTGTTGCAACGGGCCTAG
PstI                      (BclI)
```

Lieur 2

```
SalI   BamHI
TCGACTG
    GACCTAG
```

Lieur 3

```
BglII       (DraIII)
GATCTCAAACAGACAACAT
    AGTTTCTCTGTT
```

Lieur 4

```
CGCGCTCGATACTCCCTGTGACTACACTGACTTGGACACTTGGCCTTTTGCGGG
    GAGCTATGACCGACACTGATGTCACTGAACCTGTGAACCGGAAAACGCCC
BsshII
TTTGAG
AAACTCCTAG
      BamHI
```

Lieur 5

```
Hind III   EcoRI         SmaI
AGCTTGAGTCCTGAATTCGAGCTCGGTACCC
    ACTCAGGACTTAAGCTCGAGCCATGGG
```

**22.** Cellule hôte eucaryote transformée ou transfectée par un plasmide de la revendication 20 ou 21.

**23.** Procédé pour produire un polypeptide de kallikréine purifié et isolé, comprenant les étapes de :
- transfecter ou transformer des cellules hôtes par l'ADN selon la revendication 9 ;
- cultiver les cellules hôtes transfectées ou transformées pour permettre aux cellules hôtes d'exprimer le polypeptide de kallikréine ; et
- isoler la kallikréine.

**Revendications pour les Etats contractants suivants : ES, GR**

**1.** Procédé pour produire un polypeptide de kallikréine, dérivé de façon recombinante, purifié et isolé, comprenant la séquence d'acides aminés, s'étendant à partir de la terminaison N :

-1
(Ala Pro Pro Ile Gln Ser Arg)n

+1 10 20
Ile Val Gly Gly Trp Glu Cys Glu Gln His Ser Gln Pro Trp Gln Ala Ala Leu Tyr His

30 40
Phe Ser Thr Phe Gln Cys Gly Gly Ile Leu Val His Arg Gln Trp Val Leu Thr Ala Ala

50 60
His Cys Ile Ser Asp Asn Tyr Gln Leu Trp Leu Gly Arg His Asn Leu Phe Asp Asp Glu

70 80
Asn Thr Ala Gln Phe Val His Val Ser Glu Ser Phe Pro His Pro Gly Phe Asn Met Ser

90 100
Leu Leu Glu Asn His Thr Arg Gln Ala Asp Glu Asp Tyr Ser His Asp Leu Met Leu Leu

110 120
Arg Leu Thr Glu Pro Ala Asp Thr Ile Thr Asp Ala Val Lys Val Val Glu Leu Pro Thr

130 140
Gln Glu Pro Glu Val Gly Ser Thr Cys Leu Ala Ser Gly Trp Gly Ser Ile Glu Pro Glu

150 160
Asn Phe Ser Phe Pro Asp Asp Leu Gln Cys Val Asp Leu Lys Ile Leu Pro Asn Asp Glu

170 180
Cys Lys Lys Ala His Val Gln Lys Val Thr Asp Phe Met Leu Cys Val Gly His Leu Glu

190 200
Gly Gly Lys Asp Thr Cys Val Gly Asp Ser Gly Gly Pro Leu Met Cys Asp Gly Val Leu

210 220
Gln Gly Val Thr Ser Trp Gly Tyr Val Pro Cys Gly Thr Pro Asn Lys Pro Ser Val Ala

230 238
Val Arg Val Leu Ser Tyr Val Lys Trp Ile Glu Asp Thr Ile Ala Glu Asn Ser

dans laquelle n est 0 ou 1 ; et caractérisé par le fait d'être le produit de l'expression procaryote ou eucaryote d'une séquence d'ADN exogène, lequel procédé comprend l'expression de ladite séquence d'ADN dans des cellules procaryotes ou eucaryotes pour produire ledit polypeptide.

2. Procédé pour produire un polypeptide de kallikréine, dérivé de façon recombinante, purifié et isolé selon la revendication 1, dans lequel n est 0.

3. Procédé pour produire un polypeptide de kallikréine, dérivé de façon recombinante, purifié et isolé selon la revendication 1, dans lequel n est 1.

4. Procédé pour produire un polypeptide de kallikréine, dérivé de façon recombinante, purifié et isolé selon la revendication 2 et dépourvu d'association avec une protéine quelconque de mammifère.

**5.** Procédé pour produire un polypeptide de kallikréine, dérivé de façon recombinante, purifié et isolé selon la revendication 2, dans lequel la séquence d'ADN exogène est une séquence d'ADNc.

**6.** Procédé pour produire un polypeptide de kallikréine, dérivé de façon recombinante, purifié et isolé selon la revendication 2, dans lequel la séquence d'ADN exogène est une séquence d'ADN fabriqué.

**7.** Procédé pour produire un polypeptide de kallikréine, dérivé de façon recombinante, purifié et isolé selon la revendication 2, dans lequel la séquence d'ADN exogène est une séquence d'ADN génomique.

**8.** Procédé pour produire un polypeptide de kallikréine, dérivé de façon recombinante, purifié et isolé selon la revendication 1, ayant un marqueur détectable.

**9.** Procédé pour produire un ADN purifié et isolé codant pour l'expression par un hôte procaryote ou eucaryote d'un polypeptide de kallikréine pouvant être produit selon la revendication 1, lequel procédé comprend la purification et l'isolation dudit ADN.

**10.** Procédé pour produire un ADN purifié et isolé selon la revendication 9 ayant la séquence de nucléotides indiquée dans le tableau V.

**11.** Procédé pour produire un ADN purifié et isolé selon la revendication 9, dans lequel l'ADN est de l'ADNc.

**12.** Procédé pour produire un ADN purifié et isolé selon la revendication 9, dans lequel l'ADN est de l'ADN génomique.

**13.** Procédé pour produire un ADN purifié et isolé selon la revendication 9, dans lequel l'ADN est de l'ADN fabriqué.

**14.** Procédé pour produire un ADN purifié et isolé selon la revendication 13, ayant un ou plusieurs codons préférés pour l'expression dans des cellules de E. coli.

**15.** Procédé pour produire un ADN purifié et isolé selon la revendication 14, ayant la séquence de nucléotides indiquée dans le tableau VI.

**16.** Procédé pour produire une composition pharmaceutique, lequel procédé comprend la combinaison d'une quantité thérapeutiquement efficace d'un polypeptide de kallikréine pouvant être produit selon la revendication 1 et un adjuvant pharmaceutiquement acceptable.

**17.** Utilisation d'un polypeptide de kallikréine pouvant être produit selon la revendication 1 pour la fabrication d'un médicament pour fournir une thérapie de vasodilatation.

**18.** Utilisation d'un polypeptide de kallikréine pouvant être produit selon la revendication 1 pour la fabrication d'un médicament pour traiter la stérilité masculine.

**19.** Procédé pour produire une cellule hôte procaryote ou eucaryote, lequel procédé comprend la transformation ou la transfection d'une cellule procaryote ou eucaryote par de l'ADN pouvant être produit selon la revendication 9 d'une façon permettant à la cellule hôte d'exprimer un polypeptide de kallikréine pouvant être produit selon la revendication 1.

**20.** Procédé pour produire un plasmide constitué de pDSHK1, comprenant la ligature de :

a) le plasmide pDSVL digéré par BamHI et rempli aux extrémités ; et

b) la séquence d'ADN montrée dans le tableau V s'étendant de la position 738 à la position 5587.

**21.** Procédé pour produire un plasmide constitué de pDGHK-L1A, dans lequel ledit plasmide est le produit de ligature de :

a) un fragment HindIII-EcoRI constitué des nucléotides 2448 à 4362 de pBR322 ;

b) un fragment EcoRI-PstI de pMgI codant un minigène de réductase de dihydrofolate, dans lequel une délétion dans la région non traduite 3' du minigène est réalisée en retirant un fragment BglII de 556 paires de bases et en remplissant aux extrémités par une enzyme de Klenow ;
c) une séquence de terminaison SV40 bidirectionnelle constituée d'un fragment BamHI-BclI des nucléotides 2553 à 2770 d'ADN SV40, ayant un lieur-1 synthétique ajouté à l'extrémité BclI du fragment pour créer un site PstI, et ayant un lieur-2 synthétique ajouté à l'extrémité BamHI du fragment pour créer un site SalI ;
d) une séquence codant la kallikréine constituée des nucléotides 801 à 5370 comme montré dans le tableau V ayant un lieur-3 synthétique ajouté au site DraIII en position 801 et ayant un site SalI créé par insertion de GA entre les nucléotides 15 et 16 3' au codon de terminaison TGA ;
e) un promoteur du gène de la protéine de régulation du glucose chez les rats (GRP78), constitué des fragments SmaI-BsshII des nucléotides -722 à -37 de la séquence GRP78 ;
f) un lieur-4 synthétique dans lequel le site BamHI est ligaturé au site BglII du lieur-3 ; et
g) un lieur-5 synthétique, dans lequel le site SmaI est ajouté à l'extrémité SmaI de la séquence de promoteur GRP de (e) et l'extrémité HindIII ajoutée à l'extrémité HindIII de (a) ; et dans lequel les séquences des lieurs sont comme suit :

```
Lieur 1

               GCAATGGCAACAACGTTGCCCG
ACGTCGTTACCGTTGTTGCAACGGGCCTAG
PstI                        (BclI)

Lieur 2

SalI  BamHI
TCGACTG
     GACCTAG

Lieur 3

BglII        (DraIII)
GATCTCAAACAGACAACAT
     AGTTTCTCTGTT
```

```
Lieur 4
CGCGCTCGATACTCCCTGTGACTACACTGACTTGGACACTTGGCCTTTTGCGGG
     GAGCTATGACCGACACTGATGTCACTGAACCTGTGAACCGGAAAACGCCC
BsshII
TTTGAG
AAACTCCTAG
        BamHI

Lieur 5
Hind III    EcoRI            SmaI
AGCTTGAGTCCTGAATTCGAGCTCGGTACCC
     ACTCAGGACTTAAGCTCGAGCCATGGG
```

**22.** Procédé pour produire une cellule hôte eucaryote, comprenant la transformation ou la transfection d'une cellule eucaryote par un plasmide pouvant être produit selon la revendication 20 ou 21.

**23.** Procédé pour produire un polypeptide de kallikréine purifié et isolé, comprenant les étapes de :

- transfecter ou transformer des cellules hôtes par l'ADN selon la revendication 9 ;
- cultiver les cellules hôtes transfectées ou transformées pour permettre aux cellules hôtes d'exprimer le polypeptide de kallikréine ; et
- isoler la kallikréine.

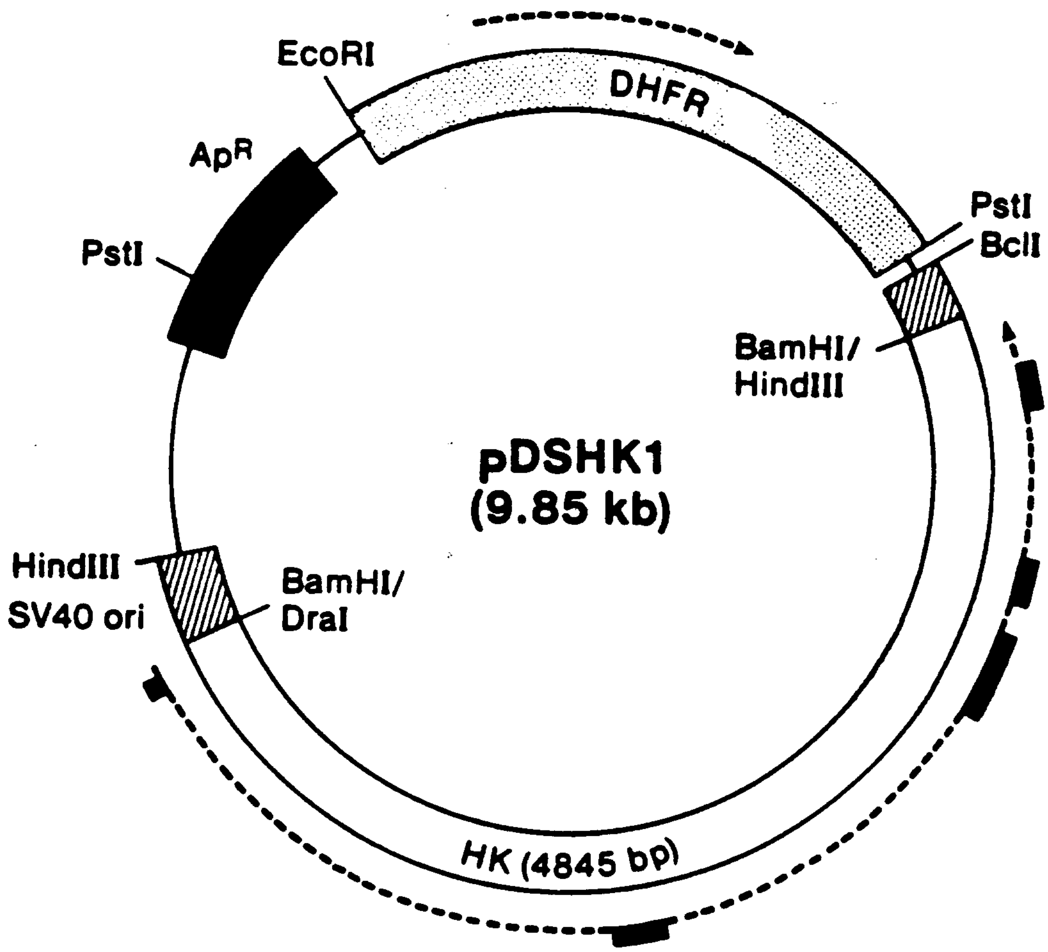
EcoRI
DHFR
ApR
PstI
PstI
BclI
BamHI/
HindIII
pDSHK1
(9.85 kb)
HindIII
SV40 ori
BamHI/
DraI
HK (4845 bp)

FIG. 1

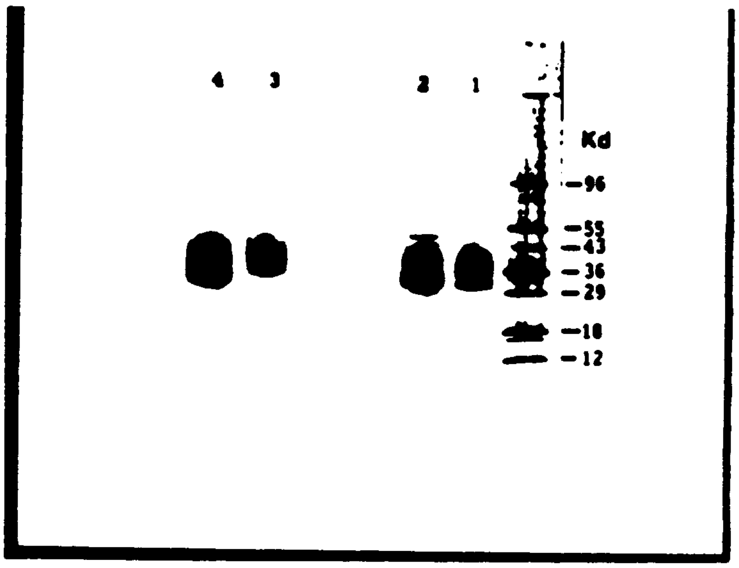


FIG. 2

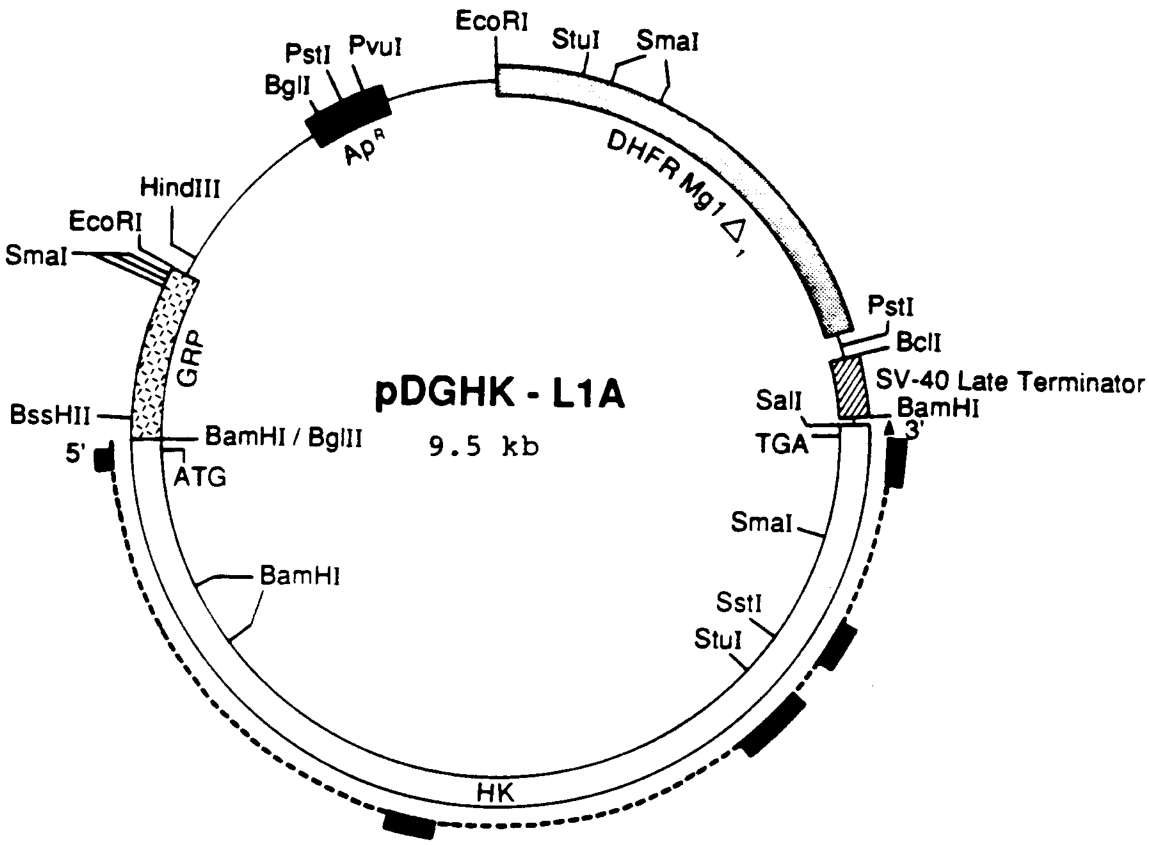
EcoRI
StuI
SmaI
PstI
PvuI
BglI
Ap^R
DHFR Mg1 Δ,
HindIII
EcoRI
SmaI
GRP
pDGHK - L1A
9.5 kb
PstI
BclI
SV-40 Late Terminator
SalI
BamHI
BssHII
BamHI / BglII
TGA
3'
5'
ATG
SmaI
BamHI
SstI
StuI
HK

FIG. 3